# EUROPEAN PATENT APPLICATION

(11) **EP 4 600 244 A1**
(43) Date of publication of application: **13.08.2025**
(21) Application number: 23876590.3
(22) Date of filing: 07.10.2023
(51) Int. Cl.: C07D 295/205, C07C 69/96, A61K 31/265, A61K 31/27, A61P 25/00, A61P 29/00

(54) **SUBSTITUTED 3-FLUOROBENZENEPROPANOATE COMPOUND, PREPARATION METHOD THEREFOR, PHARMACEUTICAL COMPOSITIONS, AND USES**

(30) Priority: 09.10.2022 CN 202211227966; 26.09.2023 CN 202311243035
(71) Applicant: Tianjin Gudui Bio-Pharm Co., Ltd., Tianjin 300384 (CN)
(72) Inventor: GAO, Qingzhi, Tianjin 300384 (CN); LIU, Shengnan, Tianjin 300384 (CN); SUN, Ziru, Tianjin 300384 (CN); LIU, Yang, Tianjin 300384 (CN); DU, Jinping, Tianjin 300384 (CN); HAN, Jianbin, Tianjin 300384 (CN); LI, Yang, Tianjin 300384 (CN); ZHANG, Shunjie, Tianjin 300384 (CN); ZHAO, Fuping, Tianjin 300384 (CN); GAO, Xiangqian, Tianjin 300384 (CN)
(74) Representative: Bandpay & Greuter
(86) International application number: PCT/CN2023/123222
(87) International publication number: WO 2024/078387

(57) **Abstract**

A substituted 3-fluorobenzenepropanoate compound represented by formula (I), an optical isomer thereof, a pharmaceutically acceptable salt thereof, a solvate (such as a hydrate) thereof, a clathrate thereof, a racemate thereof, a co-crystal thereof, an isotopic label thereof, or a nitrogen oxide thereof, as well as pharmaceutical compositions and uses thereof. The compound has significantly elevated drug activity, and can effectively treat and/or prevent inflammation, pain, fever, cancer, or senile dementia.

## Description

### TECHNICAL FIELD

The present disclosure relates to a 3-fluorobenzenepropanoate compound, a preparation method therefor, a pharmaceutical composition containing the same, and use thereof.

### BACKGROUND

Non-Steroidal Anti-Inflammatory Drug (NSAID) is a class of drugs having the antipyretic, analgesic, anti-inflammatory, and anti-rheumatic effects. There is a variety of NSAIDs with different structural formulae, but all of them have similar mechanisms of action, which are mainly to prevent arachidonic acid from generating prostacyclin (PGI2), prostaglandins (PGE1, PGE2) and thromboxane A2 (TXA2) by inhibiting the activity of cyclooxygenase (COX), ultimately achieving the anti-inflammatory and analgesic effects. At present, NSAIDs are widely used in many clinical diseases, and their total consumption worldwide is second only to antibiotics. Among treatments of various rheumatic diseases, the prescription for NSAIDs ranks first, so NSAIDs have always been one of the hot spots for research and development.

Since different NSAIDs have significantly different structures, there are obvious differences in physicochemical properties, potency, bioavailability, metabolism, half-life and other pharmacokinetics. Flurbiprofen is currently recognized as one of the NSAIDs with the most powerful anti-inflammatory and analgesic effects. Most of the commercially-available flurbiprofen formulations are oral formulations, which are prone to cause adverse effects such as gastrointestinal dysfunction. Upper gastrointestinal intolerance is also a major constraint to use of flurbiprofen. The irritation and side effects of flurbiprofen, as an arylpropionic acid-based drug, on the gastrointestinal tract are mainly caused by the carboxylic acid groups on its molecule. Scientists have structurally modified and derivatized flurbiprofen in the hope of obtaining prodrugs with less toxic side effects and higher bioavailability, thereby improving patient compliance. Flurbiprofen axetil developed by Japan Kaken Pharmaceutical Co., Ltd. is a prodrug of flurbiprofen, which boasts the advantages such as a long-lasting analgesic effect, a targeted effect, and a reduction in gastrointestinal irritation. However, because of instability in the gastrointestinal environment, flurbiprofen axetil cannot be orally administered and needs to be injected intravenously, which greatly confines its wide application.

Therefore, developing NSAIDs with higher stability, longer half-life, better efficacy and fewer toxic side effects is a demand for development of new anti-inflammatory and analgesic drugs.

### SUMMARY

The present disclosure provides a 3-fluorobenzenepropanoate compound represented by the following formula (I), or an optical isomer thereof, or a pharmaceutically acceptable salt thereof, or a solvate thereof (such as a hydrate), or an inclusion compound thereof, or a racemate thereof, or a co-crystal thereof, or an isotopically labelled compound thereof, or a nitrogen oxide thereof. The compound has a good anti-inflammatory activity and could effectively treat and/or prevent the growth and reproduction of multiple myeloma. The present disclosure further provides a preparation method for the compound, a pharmaceutical composition comprising the compound, and use of the compound.

A compound represented by formula (I), or an optical isomer thereof, or a pharmaceutically acceptable salt thereof, or a solvate thereof (optionally, the solvate is a hydrate), or an inclusion compound thereof, or a racemate thereof, or a co-crystal thereof, or an isotopically labelled compound thereof, or a nitrogen oxide thereof, 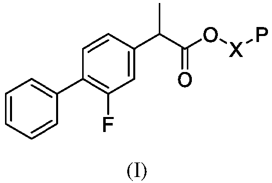 wherein:
X is alkylene;
P is 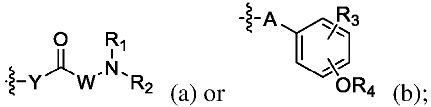
in formula (a),
Y is a single bond, -O-, -S-, or -NH-;
W is a single bond or alkylene;
R₁ and R₂ are the same or different and are each independently selected from -H, optionally substituted alkyl, and optionally substituted aryl, or R₁ and R₂ are linked to each other and jointly form, together with the nitrogen atoms to which they are linked, optionally substituted aliphatic heterocyclyl or 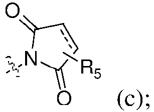
there are one or two R₃, each independently selected from -H and alkyl;
in formula (c), a dashed line indicates a single bond or null;
in formula (b),
A is a single bond, -O-(C=O)- or -O-(C=O)-alkylene;
R₃ is an optional group;
there is one or more R₃, if present, each independently alkyl, hydroxy, carboxy, hydroxy-substituted alkyl, alkoxy, or -N(R₅)(R₆), and R₃ and R₆ are the same or different and are each independently alkyl;
R₄ is selected from -H, alkyl, and alkylacyl; or
R₃ and -OR₄ are linked to each other to form an aliphatic heterocyclic ring;
the "optionally substituted" refers to being unsubstituted or substituted with one or more substituents, wherein the substituents in the "optionally substituted alkyl", "optionally substituted aryl", and "optionally substituted aliphatic heterocyclyl" are each independently selected from hydroxy, amino, carboxy, halogen, nitro, cyano, alkyl, alkylthio, alkylacyl, and hydroxy-substituted aryl;
optionally, the alkyl moieties in the "alkyl", "alkylacyl", "hydroxy-substituted alkyl", "alkoxy", and "alkylthio" are each independently C₁₋₂₀ linear or branched alkyl, optionally C₁₋₁₇ linear or branched alkyl, optionally C₁₋₁₀ linear or branched alkyl, optionally C₁₋₇ linear or branched alkyl, optionally methyl, ethyl, n-propyl, isopropyl, n-butyl, isobutyl, tert-butyl, sec-butyl, n-pentyl, isopentyl, neopentyl, tert-pentyl, n-hexyl, isohexyl, heptyl, n-octyl, n-nonyl, n-decyl, undecyl, dodecyl, tridecyl, tetradecyl, pentadecyl, or hexadecyl;
optionally, the "alkylene" is C₁₋₂₀ linear or branched alkylene, optionally C₁₋₁₇ linear or branched alkylene, optionally C₁₋₁₀ linear or branched alkylene, optionally C₁₋₈ linear or branched alkylene, optionally C₁₋₆ linear or branched alkylene, optionally C₁₋₃ linear or branched alkylene, optionally methylene, ethylene, isoethylidene, n-propylene, isopropylene, n-butylidene, isobutylidene, tert-butylidene, sec-butylidene, n-pentylidene, isopentylidene, neopentylidene, tert-pentylidene, n-hexylidene, isohexylidene, heptylidene, n-octylidene, n-nonylidene, or n-decylidene;
optionally, the aliphatic heterocyclic ring in the "aliphatic heterocyclic ring" or "aliphatic heterocyclyl" is a C₃₋₈ (preferably C₄₋₆) aliphatic heterocyclic ring containing 1 to 3 heteroatoms selected from O, N, and S on the ring, optionally dioxolane, aziridinyl, azetidinyl, tetrahydropyrrolyl, morpholinyl, piperidinyl, or piperazinyl;
optionally, the "aryl" or "hydroxy-substituted aryl" is a 6- to 10-membered monocyclic or bicyclic fused aromatic ring group; optionally phenyl or naphthyl;
optionally, the present disclosure relates to the compounds as described above and any appended definitions, wherein a 3-fluorobenzenepropanoate moiety is in an S configuration or an R configuration;
optionally, R₁ and R₂ are the same or different and are each independently selected from -H, n-propyl, isopropyl, 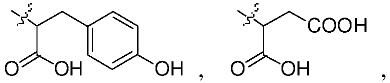 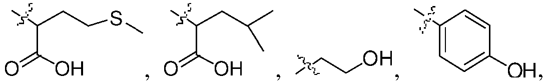 or R₁ and R₂ are linked to each other and jointly form, together with the nitrogen atoms to which they are linked, the following group: heterocyclopropan-1-yl, azetidin-1-yl, tetrahydropyrrol-1-yl, morpholin-1-yl, piperidin-1-yl, or piperazin-1-yl, 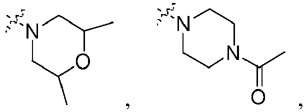 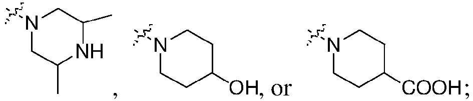
optionally, when R₃ and -OR4 are linked to each other to form an aliphatic heterocyclic ring, the aliphatic heterocyclic ring is dioxolane.

In another aspect, the present disclosure relates to the following compounds, or optical isomers thereof, or solvates thereof (such as a hydrate), or inclusion compounds thereof, or racemates thereof, or co-crystals thereof, or isotopically labelled compounds thereof, or nitrogen oxides thereof:

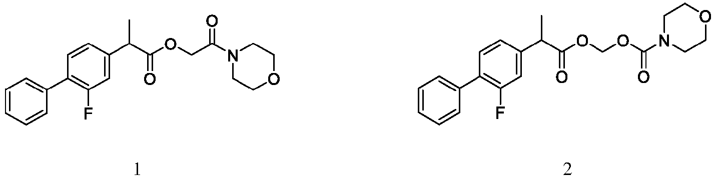

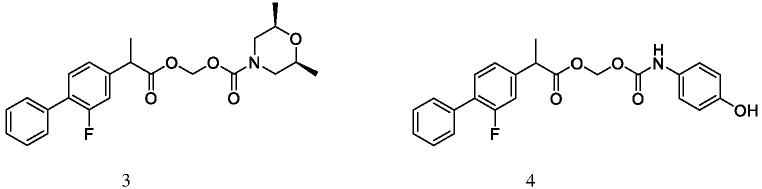

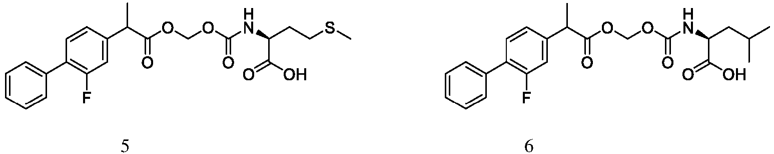

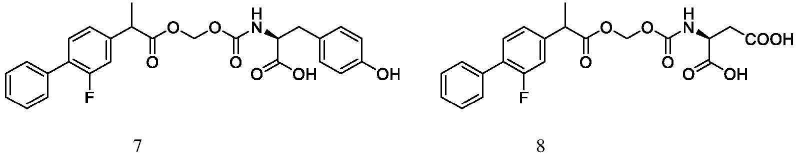

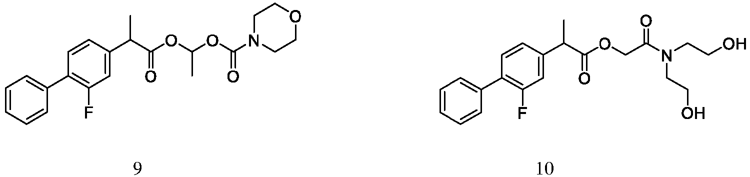

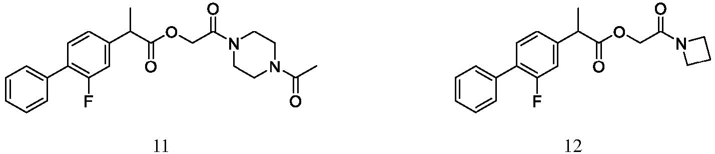

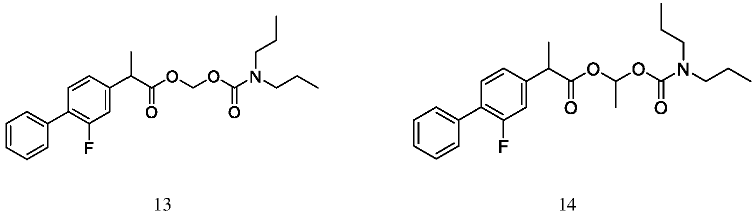

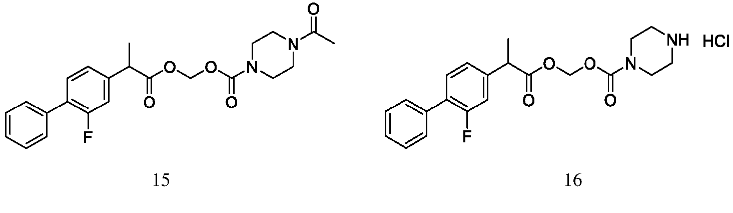

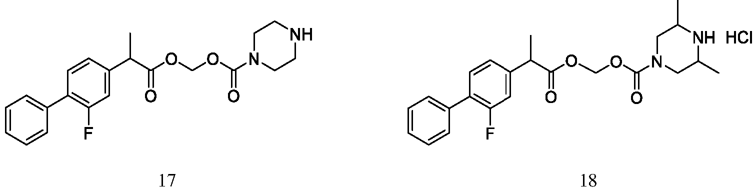

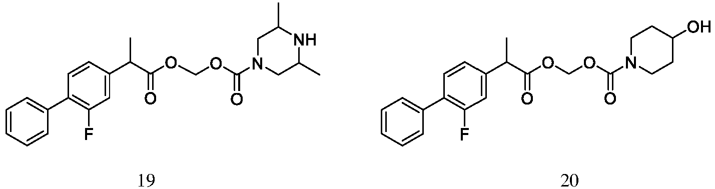

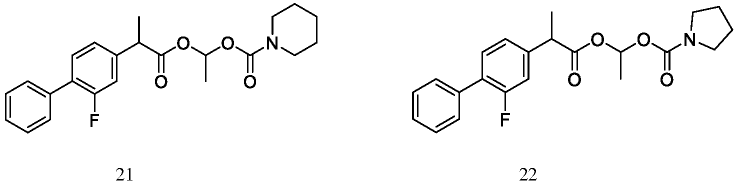

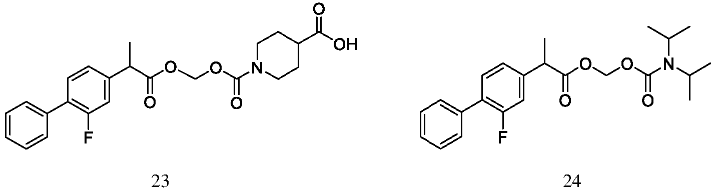

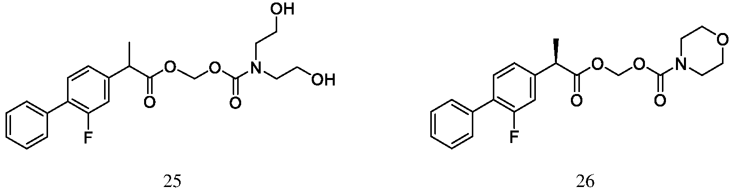

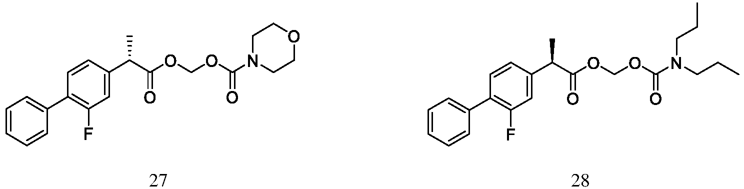

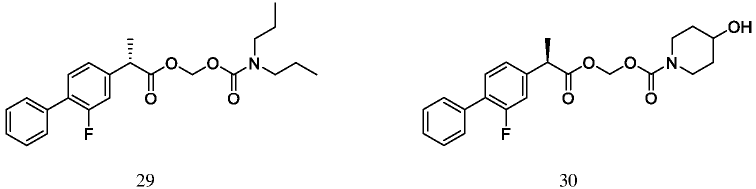

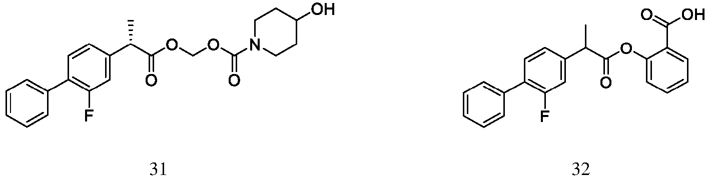

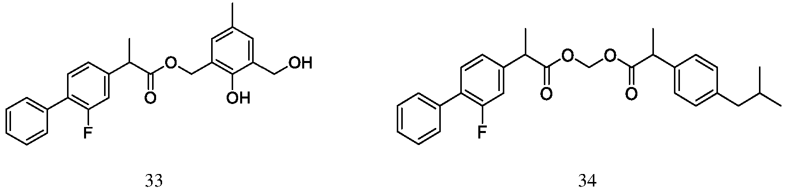

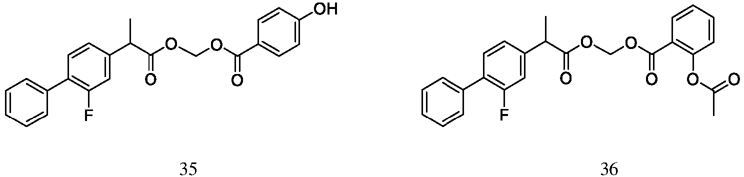

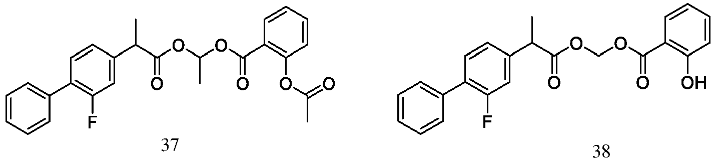

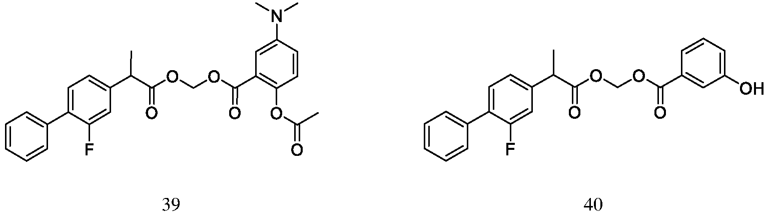

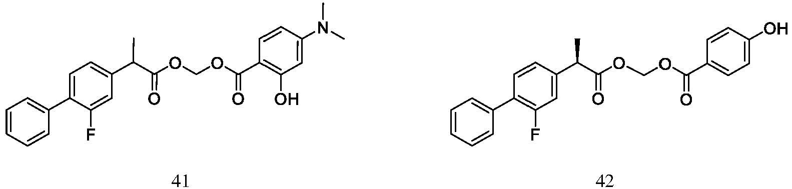

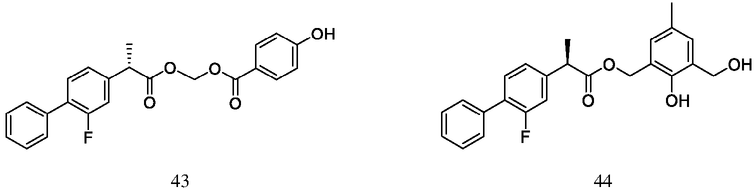

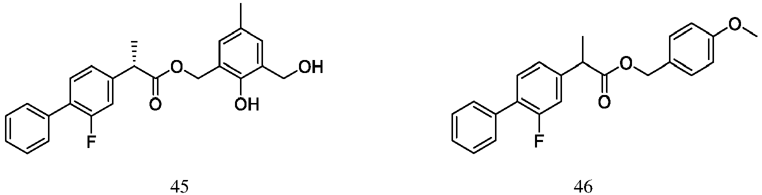

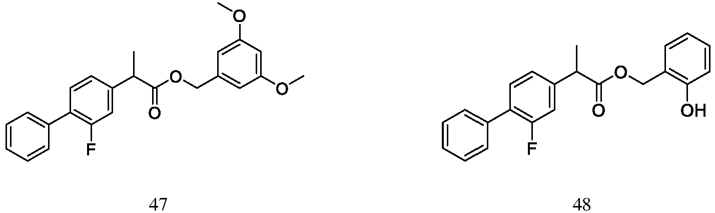

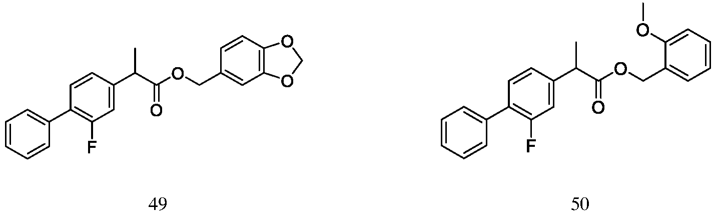

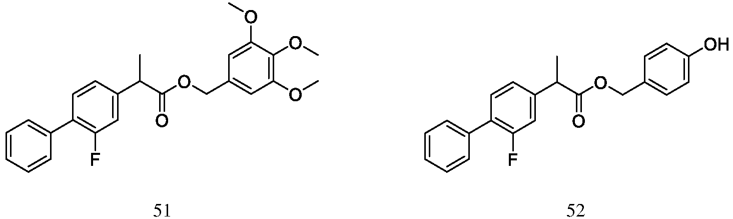

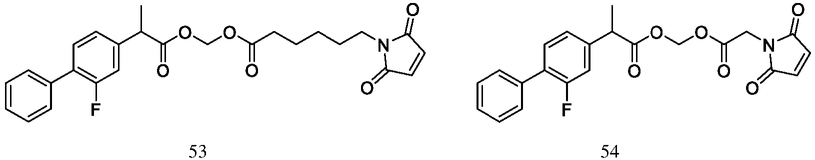

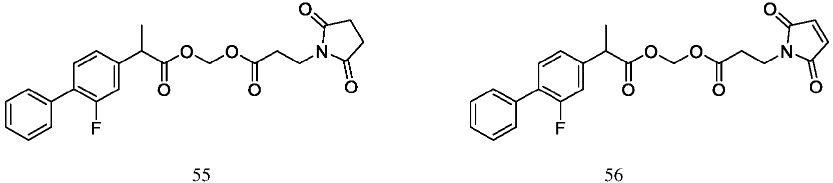

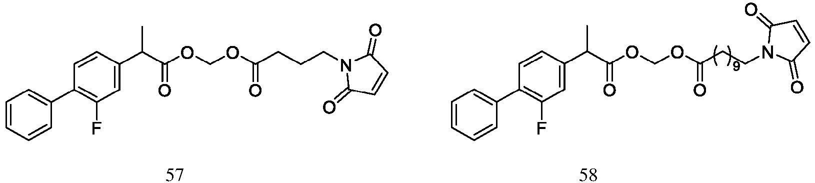

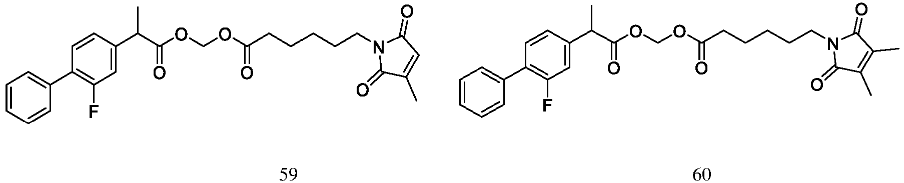

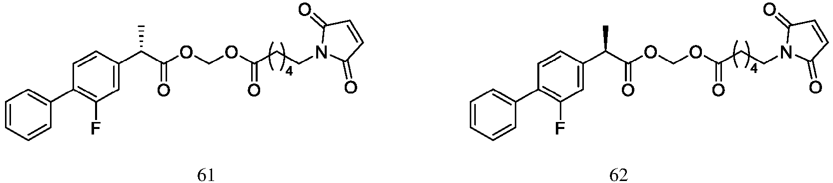

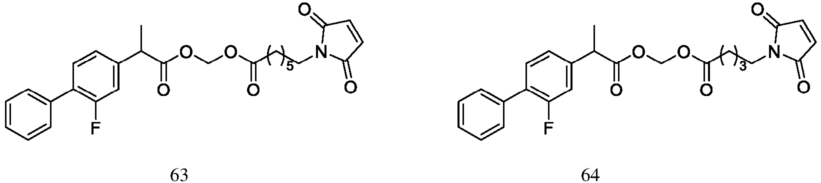

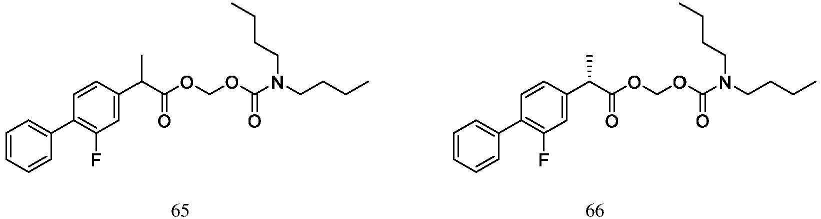

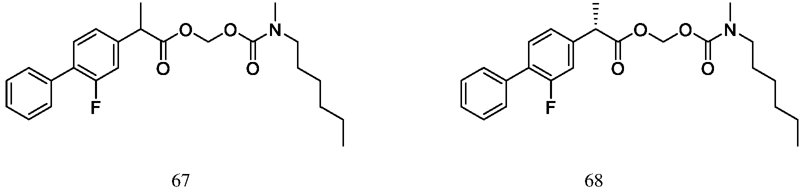

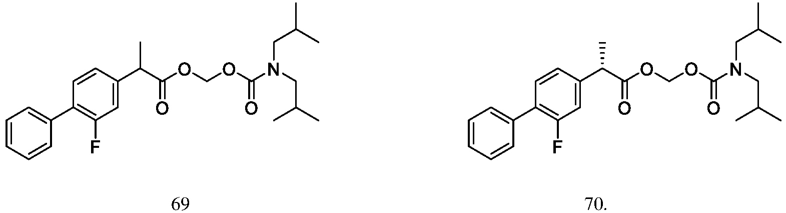

In a further aspect, the present disclosure provides a preparation method for the compound of formula (I), or the optical isomer thereof, or the pharmaceutically acceptable salt thereof, or the solvate thereof (such as a hydrate), or the inclusion compound thereof, or the racemate thereof, or the co-crystal thereof or the isotopically labelled compound thereof, or the nitrogen oxide thereof as described above,
in formula (I), when P is 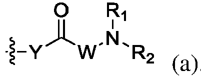 , the preparation method comprises a step of preparing formula (II) from a compound of formula (A) and a compound of formula (B) by method A:
method A: preparing formula (II) by subjecting formula (A) and formula (B) to a substitution reaction; 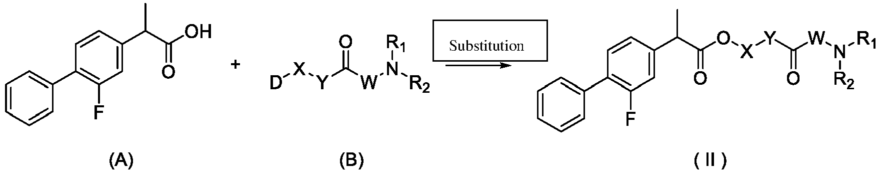 wherein
formula (A) is an R configuration, an S configuration or a racemate;
in formula (B) and formula (II), X, Y, W, R₁ and R₂ are as described in formula (I);
in formula (B), D is selected from Cl, Br, I, OTs, and OMs;
each Y is -O-, -S-, or -NH-, wherein R₁ and R₂ are as described in formula (I).

Optionally, formula (II) is prepared by subjecting formula (A) and formula (B) to a substitution reaction in a suitable solvent at a suitable temperature under catalysis of a base.

Optionally, in the method A, the base used for the substitution reaction is one or more selected from pyridine or triethylamine or N,N-diisopropylethylamine or 1,8-diazabicyclo[5.4.0]undec-7-ene or potassium carbonate or cesium carbonate or sodium hydride or lithium bis(trimethylsilyl)amide; optionally, the solvent for the substitution reaction is one or more selected from DMF or DMAC or dimethyl sulfoxide or NMP or DCM or tetrahydrofuran or acetonitrile or ethyl acetate or isopropyl acetate or dioxane or acetone; optionally, the temperature for the substitution reaction is 0°C to 100°C.

Optionally, formula (B) may be prepared by, but not limited to, one of the following steps 1 to 3:
step 1: 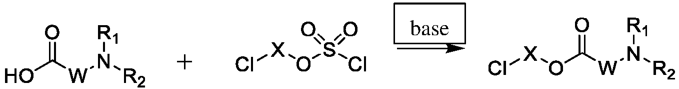 at room temperature, a saturated sodium bicarbonate solution and tetrabutylammonium hydrogen sulfate at a catalytic amount are added to a solution of an acid in dichloromethane that is being vigorously stirred, and then chloromethyl chlorosulfonate is added; after the reaction is completed, the reaction solution is layered, and the organic phases are collected, dried over anhydrous sodium sulfate, evaporated to dryness via rotary evaporation under reduced pressure, and purified by column chromatography to yield the compound of formula (B).
And/or step 2: 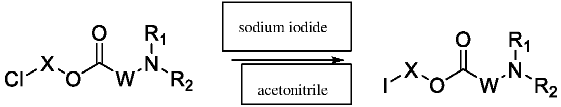 a chlorination reagent and sodium iodide (or potassium iodide) are refluxed in acetonitrile (or acetone); after the reaction is completed, the solvent is removed under reduced pressure, and an organic solvent and water are added; the reaction solution is layered, and the organic phases are collected and dried over anhydrous sodium sulfate; and the solvent is removed under reduced pressure to yield a corresponding iodinated compound of formula (B).

Optionally, when W is absent, formula (B) is synthesized by step 3;
or step 3: 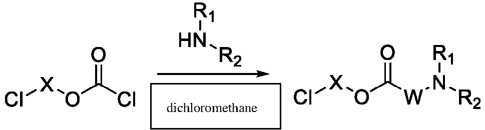 at 0°C, a corresponding secondary amine is added to a solution of chloromethyl chloroformate in dichloromethane; after the reaction is completed, the reaction solution is filtered, and the organic phases are washed with 1N hydrochloric acid and dried over anhydrous sodium sulfate; and the solvent is removed under reduced pressure to yield the compound of formula (B).

Or when W is a single bond, the preparation method comprises preparing formula (II) from formula (A), formula (C) and formula (M) by method B;
method B: ① preparing formula (D) from formula (A) and formula (C) by a condensation reaction; ② preparing formula (E) by de-protecting formula (D); and ③ preparing formula (II) by condensing formula (E) with formula (M); 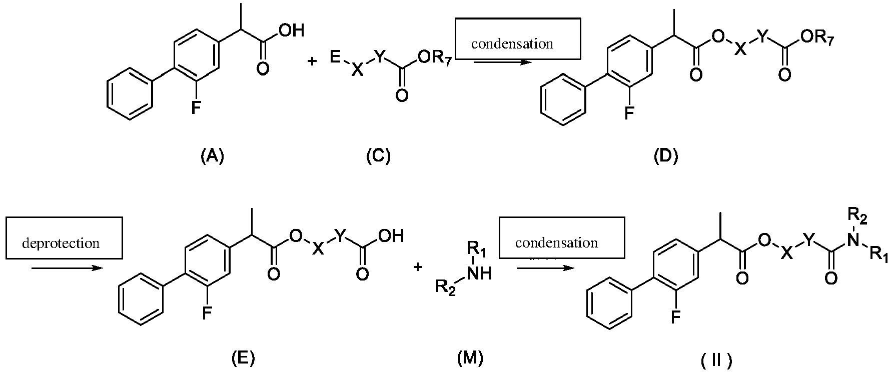 wherein
Y is a single bond, and X, R₁ and R₂ are as described in formula (I);
formula (A) is an R configuration, an S configuration or a racemate;
in formula (C), R₇ is methyl, ethyl, tert-butyl, benzyl, or p-methoxybenzyl; and E is -OH.

Or when P is 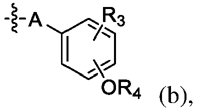 the preparation method comprises preparing formula (III) from a compound of formula (A) and a compound of formula (F) by method C;
method C: preparing formula (III) by subjecting formula (A) and formula (F) to a condensation reaction; 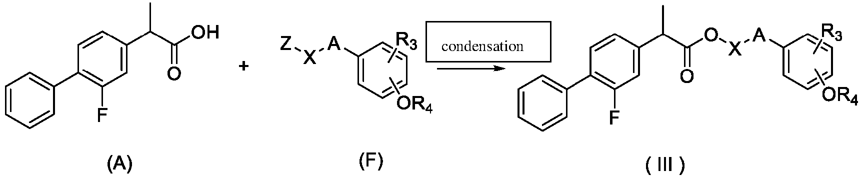 wherein
in formula (F), A is a single bond, and X, R₃ and R₄ are as described in formula (I);
in formula (F), Z is selected from -OH, -NH₂, -SH, and -NH-;
optionally, in the method B and method C, a condensing agent for the condensation reaction is one or more selected from DCC, DIC, EDCI, HATU, HBTU, CDI, HCTU, TBTU, TSTU, TNTU, HAPyU, HBPyU, BOP, PyBOP, PyAOP, DPPCl, DECP, DPPA, MPTA, or BOPCl, optionally a solvent for the condensation reaction is one or more selected from dichloromethane, dimethyl sulfoxide, N,N-dimethylformamide, hexamethylphosphoric triamide, or acetonitrile; optionally, the temperature for the condensation reaction is 0°C to 100°C.

Or the preparation method comprises preparing a compound of formula (III) from formula (A) by method D:
method D: 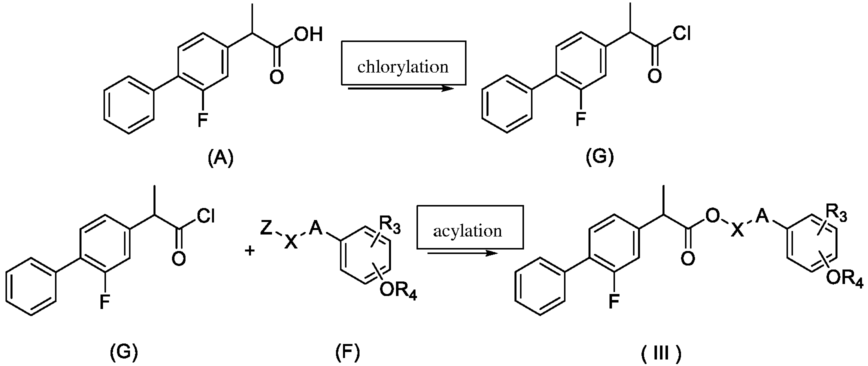 wherein
in formula (F), A is a single bond, and X, R₃, R₄ and Z are as described above;
or the preparation method comprises preparing a compound of formula (III) from formula (A) and formula (H) by method E:
   method E: 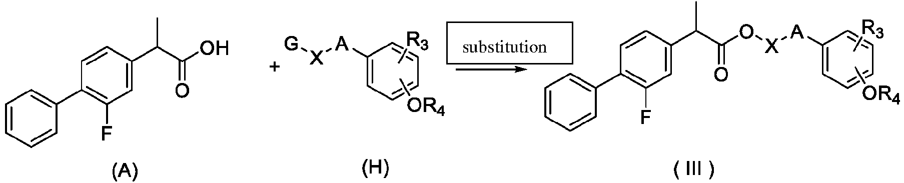 wherein
   in formula (H), A is -O-(C=O)-; G is Cl, Br, I, OMs, or OTs; and X, R₃ and R₄ are as described in formula (I);
   optionally, the formula (B) in method A may be prepared by (but not limited to) one of methods a to e:
      when D in the formula (B) is Cl, formula (B1) is prepared by the following method a:
      at low temperature, a base is added to formula (M), and then chloromethyl chloroformate or chloroethyl chloroformate is added; after the reaction is completed, the organic phase is eluted with 1N citric acid, dried over anhydrous sodium sulfate, and distilled under reduced pressure to vield formula (B1); 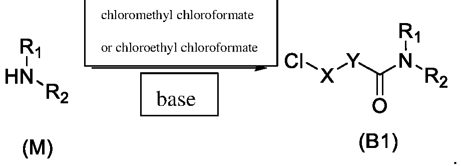
      Or when D in the formula (B) is Br, formula (B2) is prepared by the following method b: chloromethyl reagent and sodium bromide (or potassium bromide) are refluxed in acetonitrile (or acetone); after the reaction is completed, the solvent is removed under reduced pressure, and an organic solvent and water are added; the reaction solution is layered, and the organic phases are collected, dried over anhydrous sodium sulfate, and distilled under reduced pressure to yield a corresponding brominated compound of formula (B2); 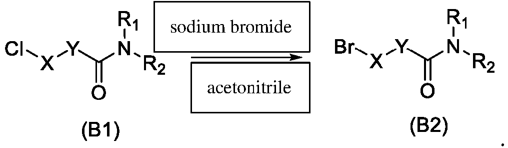
      Or when D in the formula (B) is I, formula (B3) is prepared by the following method c:
         chloromethyl reagent and sodium iodide (or potassium iodide) are refluxed in acetonitrile (or acetone); after the reaction is completed, the solvent is removed under reduced pressure, and an organic solvent and water are added; the reaction solution is layered, and the organic phases are collected, dried over anhydrous sodium sulfate, and distilled under reduced pressure to yield a corresponding iodinated compound of formula (B3); 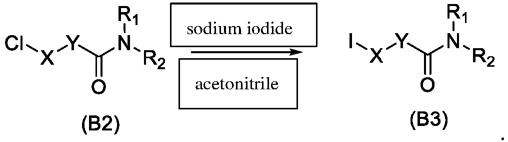
         Or when D in the formula (B) is OMs, formula (B4) is prepared by the following method d: 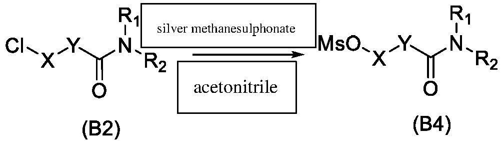
         chloromethyl reagent and silver methanesulphonate are refluxed in acetonitrile; after the reaction is completed, the solvent is removed under reduced pressure, and an organic solvent and water are added; the reaction solution is layered; and the organic phases are collected, dried over anhydrous sodium sulfate, and distilled under reduced pressure to yield a corresponding methylsulfonyloxy substituted compound of formula (B4).
         Or when D in the formula (B) is OTs, formula (B5) is prepared by the following method e: 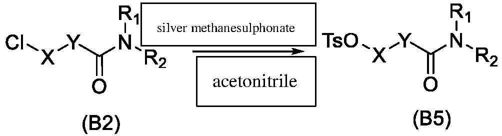
         chloromethyl reagent and silver p-toluenesulfonate are refluxed in acetonitrile; after the reaction is completed, the solvent is removed under reduced pressure, and an organic solvent and water are added; the reaction solution is layered; and the organic phases are collected, dried over anhydrous sodium sulfate, and distilled under reduced pressure to yield a corresponding p-phenylsulfonyloxy substituted compound of formula (B5).

Optionally, the formula (H) in method E is prepared by (but not limited to) one of methods f to k:
When G is Cl in the formula (H), formula (H1) is prepared by the following method f: 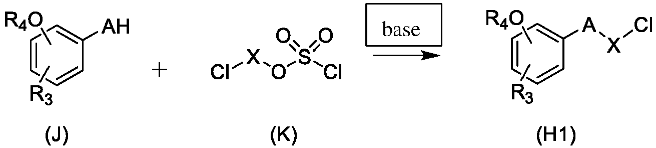
at room temperature, a saturated sodium bicarbonate solution and tetrabutylammonium hydrogen sulfate at a catalytic amount are added to a solution of formula (J) in dichloromethane acid, and then added to formula (K); after the reaction is completed, the reaction solution is layered; and the organic phases are collected, dried over anhydrous sodium sulfate, evaporated to dryness via rotary evaporation under reduced pressure, and purified by column chromatography to yield formula (H1).

Or formula (H1) is prepared by the following method g:

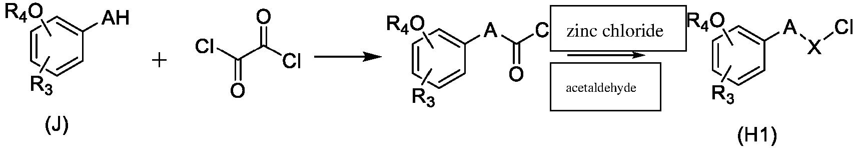

Or when G is Br in the formula (H), formula (H2) is prepared by the following
method h: 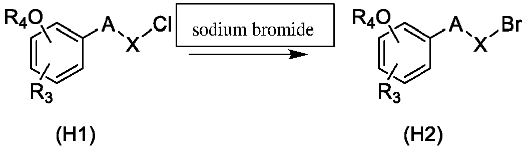 formula (H1) and sodium bromide (or potassium bromide) are refluxed in acetonitrile (or acetone); after the reaction is completed, the solvent is removed under reduced pressure, and an organic solvent and water are added; the reaction solution is layered; and the organic phases are collected, dried over anhydrous sodium sulfate, and distilled under reduced pressure to yield formula (H2).

Or when G is I in the formula (H), formula (H3) is prepared by the following method i: 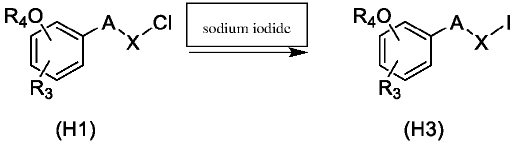 formula (H1) and sodium iodide (or potassium iodide) are refluxed in acetonitrile (or acetone); after the reaction is completed, the solvent is removed under reduced pressure, and an organic solvent and water are added; the reaction solution is layered; and the organic phases are collected, dried over anhydrous sodium sulfate, and distilled under reduced pressure to yield formula (H3).

Or when G is OMs in the formula (H), formula (H4) is prepared by the following method j: 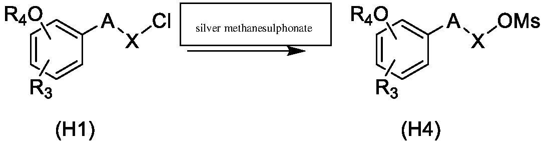 formula (H1) and silver methanesulphonate are refluxed in acetonitrile (or acetone); after the reaction is completed, the solvent is removed under reduced pressure, and an organic solvent and water are added; the reaction solution is layered; and the organic phases are collected, dried over anhydrous sodium sulfate, and distilled under reduced pressure to yield formula (H4).

Or when G is OTs in the formula (H), formula (H5) is prepared by the following method k: 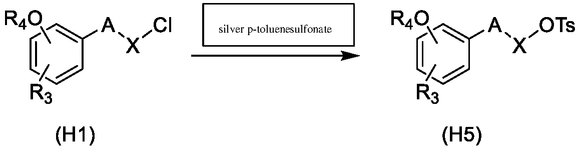 formula (H1) and silver p-toluenesulfonate are refluxed in acetonitrile (or acetone); after the reaction is completed, the solvent is removed under reduced pressure, and an organic solvent and water are added; the reaction solution is layered; and the organic phases are collected, dried over anhydrous sodium sulfate, and distilled under reduced pressure to yield formula (H5).

The pharmaceutically acceptable salt is an inorganic acid salt or an organic acid salt, preferably selected from hydrochloric acid, sulfuric acid, phosphoric acid, nitric acid, methanesulfonic acid, tartaric acid, formic acid, acetic acid, salicylic acid, citric acid, succinic acid, fumaric acid, or benzoic acid.

In a further aspect, the present disclosure provides a pharmaceutical composition, comprising the compound represented by formula (I), or the optical isomer thereof, or the pharmaceutically acceptable salt thereof, or the solvate thereof (such as a hydrate), or the inclusion compound thereof, or the racemate thereof, or the co-crystal thereof or the isotopically labelled compound thereof, or the nitrogen oxide thereof as described above, and a pharmaceutically acceptable excipient.

Optionally, the pharmaceutically acceptable excipient is selected from fillers, disintegrants, lubricants, glidants, effervescent agents, flavoring agents, preservatives, solubilizers, cosolvents, antioxidants, anti-photolysis agents, pH regulators, emulsifiers, antibacterial preservatives, topical analgesics, complexing agents, non-aqueous solvents, coating materials or other vehicles.

Optionally, as the pharmaceutically acceptable excipient, the fillers comprise a composition of one or more of lactose, mannitol, and calcium carbonate; the binders comprise a composition of one or more of sucrose, starch, povidone, and sodium carboxymethyl cellulose; the disintegrants comprise a composition of one or more of starch, cross-linked povidone, cross-linked sodium carboxymethyl cellulose, and effervescent disintegrants; the non-aqueous solvents comprise a composition of one or more of soybean oil, castor oil, and peanut oil; the solubilizers comprise a composition of one or more of Tween 80, Tween 60, and poloxamer 68; the cosolvents comprise a composition of one or more of sodium benzoate, sodium salicylate, and sodium p-aminobenzoate.

Optionally, a mode of administration of the pharmaceutical composition comprises oral administration (e.g., oral cavity), sublingual administration, parenteral administration (e.g., intramuscular, intravenous or subcutaneous administration), rectal administration (e.g., by suppositories or lotions), transdermal administration (skin electroporation, transdermal preparations, etc., such as creams, gels, paints, and transdermal patches) or inhalation administration (e.g., aerosols), and comprises administration of tablets, suspensions and the like in a solid, liquid or gaseous dosage form. The pharmaceutical composition may be administered in a single unit dose form or in an *ad libitum* single dose under continuous treatment. The therapeutic composition may further be in the form of an oil emulsion or dispersion in combination with a lipophilic salt such as pamoic acid, or in the form of a biodegradable sustained-release composition for use in subcutaneous or intramuscular administration.

Optionally, the pharmaceutical composition may be prepared into a solid oral formulation, a liquid oral formulation, an injection or a transdermal formulation. The solid and liquid oral formulations comprise: tablets, dispersible tablets, sugar-coated tablets, granules, dry powders, capsules, syrups, and solutions. The injection comprises: small injections, large volume parenteral, lyophilized powder injections, and the like. The transdermal formulation comprises: ointments, plasters, liniments, aerosols, traditional pastes, adhesive dispersion patches, peripheral adhesive skeleton patches, reservoir patches, cataplasms, and the like.

In a further aspect, the present disclosure provides use the compound represented by formula (I), or the optical isomer thereof, or the pharmaceutically acceptable salt thereof, or the solvate thereof (such as a hydrate), or the inclusion compound thereof, or the racemate thereof, or the co-crystal thereof or the isotopically labelled compound thereof, or the nitrogen oxide thereof as described above, or the pharmaceutical composition as described above in the preparation of a medicament for preventing and/or treating inflammation, pain, fever, cancer, Alzheimer's disease, etc.; preferably, the inflammation is selected from rheumatoid arthritis, osteoarthritis, ankylosing spondylitis, scapulohumeral periarthritis, myotenositis and tenosynovitis, peritendinitis, lateral epicondylitis (tennis elbow), postoperative anti-inflammation, and swelling and inflammation caused by injury; preferably, the pain is selected from mild to moderate pain, pain after trauma or strain, dysmenorrhea and postoperative pain, toothache and cancer pain, acute pain in adults, etc.; the fever comprises fever caused by common cold or influenza; the cancer is selected from gastric cancer, esophageal cancer, multiple myeloma, brain glioma, and lung cancer. Preferably, cells of the cancer comprise human esophageal carcinoma cell Eca-109, human multiple myeloma cell MM.1S, human gastric carcinoma cell NUGC-4, human brain astrocyte U87, human glioma cell U251, and large cell lung carcinoma cell H460.

In a further aspect, the present disclosure provides use of the compound represented by formula (I), or the optical isomer thereof, or the pharmaceutically acceptable salt thereof, or the solvate thereof (such as a hydrate), or the inclusion compound thereof, or the racemate thereof, or the co-crystal thereof or the isotopically labelled compound thereof, or the nitrogen oxide thereof as described above, or the pharmaceutical composition as described above in combination with additional one or more active drugs ("the second active compounds") in the preparation of a medicament for preventing and/or treating inflammation, pain, fever, cancer or Alzheimer's disease; preferably, the inflammation is selected from rheumatoid arthritis, osteoarthritis, ankylosing spondylitis, scapulohumeral periarthritis, myotenositis and tenosynovitis, peritendinitis, lateral epicondylitis (tennis elbow), postoperative anti-inflammation, and swelling and inflammation caused by injury; the pain is selected from mild to moderate pain, pain after trauma or strain, dysmenorrhea and postoperative pain, toothache and cancer pain, acute pain in adults, etc.; the fever comprises fever caused by common cold or influenza; the cancer is selected from gastric cancer, esophageal cancer, multiple myeloma, brain glioma, and lung cancer. Preferably, cells of the cancer comprise human esophageal carcinoma cell Eca-109, human multiple myeloma cell MM.1S, human gastric carcinoma cell NUGC-4, human brain astrocyte U87, human glioma cell U251, and large cell lung carcinoma cell H460. Examples of the second active compounds according to the present disclosure may be one or more selected from the following substances: sufentanil, dexmedetomidine, formoterol, isoproterenol, salbutamol, bambuterol, procaterol, fenoterol, arformoterol, tulobuterol, clenbuterol, salmeterol, salmeterol casone, terbutaline, orciprenaline, chlorprenaline, etc.

As used herein, unless otherwise indicated, the term "treatment" refers to alleviation or reduction of the severity of symptoms associated with the disease or condition being treated, such as pain, inflammation or rheumatism. The term "prevention" includes inhibition of the symptoms of a particular disease or condition, such as pain, inflammation or rheumatism.

The compound of the present disclosure can exist at an isotope trace level or an isotopically enriched level and contain one or more atoms whose atomic weight or mass number differs from that of the most abundant atom found in nature. The isotope can be radioactive or non-radioactive. Isotopes of atoms such as hydrogen, carbon, phosphorus, sulfur, fluorine, chlorine, and iodine include, but are not limited to: ²H, ³H, ¹³C, ¹⁴C, ¹⁵N, ¹⁸O, ³²P, ³⁵S, ¹⁸F, ³⁶Cl, and ¹²⁵I. Compounds containing those and/or other isotopes of other atoms are within the scope of the present disclosure.

As used herein, the term "optical isomer" refers to the substances that share exactly the same molecular structure and similar physical and chemical properties but have different optical activities. It includes optically isomeric mixtures in any proportion. The compound of formula (I) may contain one or more asymmetric carbon atoms, and may exist in the form of an optically pure enantiomer, such as an enantiomeric mixture of racemates, an optically pure diastereomer, a mixture of diastereoisomers, a racemate of diastereomers, or a mixture of racemates of diastereomers. Optically active form may be obtained, for example, by resolution of racemates, asymmetric synthesis or asymmetric chromatography (chromatography using chiral adsorbents or eluents). The present disclosure includes all of these forms.

As used herein, the term "solvate" refers to a compound that is further bound by a chemical or non-chemical amount of a solvent via non-covalent intermolecular forces. For example, when the solvent is water, the solvate is a hydrate.

### Abbreviations

BOP: tri(dimethylamino)benzotriazol-1-yloxyphosphonium hexafluorophosphate (R.20.6, 21.11.2023)
BOPCl: bis(2-oxo-3-oxazolidinyl)phosphinic chloride (R.20.6, 21.11.2023)
DCC: dicyclohexyl carbodiimide
DECP: diethyl cyanophosphonate (R.20.6, 21.11.2023)
DIC: N,N'-diisopropylcarbodiimide
EDCI: 1-ethyl-(3-dimethylaminopropyl)carbodiimide hydrochloride
HAPyU: O-(7-azabenzotriazol-1-yl)-bis(tetrahydropyrrolyl)carbonium hexafluorophosphate (R.20.6, 21.11.2023)
HATU: 2-(7-azabenzotriazole)-N,N,N',N'-tetramethyluronium hexafluorophosphate
HBTU: benzotriazole-N,N,N',N'-tetramethyluronium hexafluorophosphate
HBPyU: O-(benzotriazol-1-yl)-bis(tetrahydropyrrolyl)carbonium hexafluorophosphate (R.20.6, 21.11.2023)
CDI: N,N-carbonyldiimidazole
HCTU: O-(6-chlorobenzotriazol-1-yl)-1,1,3,3-tetramethyluronium tetrafluoroborate
MPTA: thiodimethylphosphoryl azide (R.20.6, 21.11.2023)
TBTU: benzotriazole-N,N,N',N'-tetramethyluronium hexafluorophosphate borate
TSTU: O-(N-succinimidyl)-1,1,3,3-tetramethyluronium tetrafluoroborate
DMAP: 4-dimethylaminopyridine
DMF: N,N-dimethylformamide
DMAC: N,N-dimethylacetamide
DCM: dichloromethane
DIPEA: N,N-diisopropylethylamine
DPPCl: diphenylphosphinic chloride (R.20.6, 21.11.2023)
DPPA: diphenyl azidophosphate (R.20.6, 21.11.2023)
NMP: N-methylpyrrolidone
PyAOP: (7-azabenzotriazole-1-oxy)tripyrrolylphosphonium hexafluorophosphate (R.20.6, 21.11.2023)
PyBOP: benzotriazol-1-yl-oxytripyrrolidino-phosphonium hexafluorophosphate (R.20.6, 21.11.2023)
TNTU: 2-(endo-5-norbornene-2,3-dicarboximide)-1,1,3,3-tetramethyluronium tetrafluoroborate
Eca-109: human esophageal carcinoma cell
MM.1S: human multiple myeloma cell
NUGC-4: human gastric carcinoma cell
U87: human brain astrocyte
U251: human glioma cell
H460: large cell lung carcinoma cell

### BRIEF DESCRIPTION OF THE DRAWINGS

FIG. 1 is a diagram showing changes in body weight of rats in each of groups during administration in Test Example 4 (n=8, Mean ± SD).
FIG. 2 is a diagram showing the arthritis scores of rats with CIA after 14-day intervention with each of test drugs in Test Example 4 (n=8, Mean ± SD).
FIG. 3 is a diagram showing the toe volume changes in the left hind limbs of rats with CIA after 14-day intervention with each of test drugs in Test Example 4 (n=8, Mean ± SD).
FIG. 4 shows the mechanical withdrawal thresholds of the left lower limbs of rats in each of groups after 13-day intervention with each of test drugs in Test Example 4 (Mean ± SD).

### DETAILED DESCRIPTION

Unless otherwise defined, all technical and scientific terms used herein have the same meanings as those commonly understood by a person skilled in the art. If there are multiple definitions for a term used herein, the definition defined here will prevail unless otherwise stated.

The examples of the present disclosure will be described in detail below, but the examples provided herein are not intended to limit the present disclosure in any way.

### Examples

### General Synthetic Method 1:

Method B was adopted for synthesis.

### Step 1:

Formula (A) (1.0 eq.), tert-butyl 2-glycolate (1.1 eq.), EDCI (1.5 eq.), and DMAP (0.1 eq.) were added to 5 mL of DMF, and stirred at room temperature for 12 h. Ethyl acetate was added. Thereafter, the organic phases were washed with a saturated aqueous ammonium chloride solution, water, and a saturated aqueous sodium chloride solution in sequence. The organic phases were collected, dried over anhydrous sodium sulfate, and filtered to obtain a filtrate. The filtrate was evaporated to dryness under reduced pressure. The product was dissolved into DCM, and trifluoroacetic acid (2 mL) was added, stirred for 4 h, and then concentrated under reduced pressure to yield a white solid.

### Step 2:

The above product (1.0 eq.), formula (M) (1.1 to 2 eq.), HATU (1.2 to 1.5 eq.), and DIPEA (5.0 eq.) were added to 5 mL of DMF, and stirred at room temperature for 4 h. After the reaction was completed, ethyl acetate was added. Thereafter, the organic phases were washed with a saturated aqueous ammonium chloride solution, water, and a saturated aqueous sodium chloride solution in sequence. The organic phases were collected, dried over anhydrous sodium sulfate, concentrated under reduced pressure, and purified by silica gel column chromatography to yield the product.

### Example 1: Preparation of Compound 1

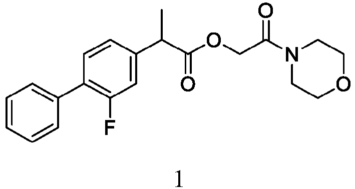

General Synthetic Method 1 was employed for preparation to yield a colorless liquid with a yield of 47.9%.

¹H NMR (400 MHz, DMSO-*d*₆) δ 7.57 - 7.44 (m, 5H), 7.43 - 7.38 (m, 1H), 7.34 - 7.26 (m, 2H), 4.90 - 4.77 (m, 2H), 4.00 (q, *J =* 7.2 Hz, 1H), 3.55 (t, *J =* 4.8 Hz, 4H), 3.41 (d, *J* = 5.0 Hz, 2H), 3.35 (s, 2H), 1.47 (d, *J =* 7.1 Hz, 3H).

### Example 2: Preparation of Compound 10

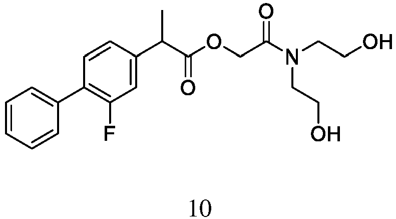

General Synthetic Method 1 was employed for preparation to yield a colorless liquid with a yield of 41.3%.

¹H NMR (400 MHz, DMSO-*d*₆) δ 7.57 - 7.52 (m, 2H), 7.52 - 7.45 (m, 3H), 7.43 - 7.38 (m, 1H), 7.34 - 7.26 (m, 2H), 4.90 (d, *J* = 5.7 Hz, 2H), 4.00 - 3.94 (m, 1H), 3.67 - 3.58 (m, 1H), 3.52 (q, *J* = 5.3 Hz, 2H), 3.46 (q, *J* = 5.9 Hz, 2H), 3.33 (s, 4H), 3.18 - 3.10 (m, 1H), 1.46 (d, *J* = 7.1 Hz, 3H).

### Example 3: Preparation of Compound 11

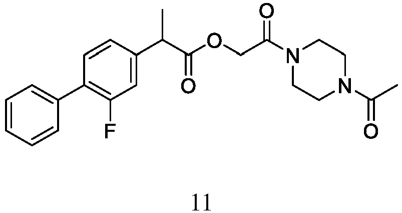

General Synthetic Method 1 was employed for preparation to yield a colorless liquid with a yield of 52.6%.

¹H NMR (400 MHz, DMSO-*d*₆) δ 7.56 - 7.45 (m, 5H), 7.43 - 7.38 (m, 1H), 7.35 - 7.26 (m, 2H), 4.94 - 4.80 (m, 2H), 4.00 (q, *J* = 7.1 Hz, 1H), 3.42 (m, 8H), 2.01 (s, 3H), 1.47 (d, *J* = 7.1 Hz, 3H).

### Example 4: Preparation of Compound 12

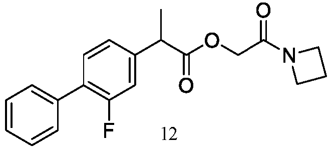

General Synthetic Method 1 was employed for preparation to yield a colorless liquid with a yield of 59.6%.

¹H NMR (400 MHz, DMSO-*d*₆) δ 7.57 - 7.45 (m, 5H), 7.43 - 7.37 (m, 1H), 7.34 - 7.23 (m, 2H), 4.61 - 4.48 (m, 2H), 4.09 (td, *J =* 7.8, 3.5 Hz, 2H), 3.99 (q, *J =* 7.1 Hz, 1H), 3.87 (t, *J =* 7.7 Hz, 2H), 2.21 (p, *J =* 7.6 Hz, 2H), 1.45 (d, *J* = 7.1 Hz, 3H).

### General Synthetic Method 2:

Method A was adopted for synthesis.

### Step 1:

Formula (M) (1.0 eq.) or protective group (Boc, tert-butyl)-protected formula (M) (1.0 eq.) was dissolved into DCM. At 0°C, chloromethyl chloroformate or 1-chloroethyl chloroformate (1.1 to 1.5 eq.) and pyridine (1.6 eq.) or triethylamine (0.6 to 1.6 eq.) were added dropwise, and stirred at -78°C to room temperature for 1 h. After the reaction was completed, DCM and water were added for extraction. The organic phases were collected, dried over anhydrous sodium sulfate, filtered, and then concentrated under reduced pressure to yield a transparent colorless compound.

### Step 2:

Formula (A) (1.0 eq.) was dissolved into anhydrous DMF, and potassium carbonate (1.6 to 2.0 eq.) was added. Afterwards, the above product was dissolved into DMF, and added to the reaction solution dropwise, heated to 40°C to 50°C, and stirred for 4 h. After the reaction was completed, ethyl acetate was added. Thereafter, the organic phases were washed with a saturated aqueous ammonium chloride solution, water, and a saturated aqueous sodium chloride solution in sequence. The organic phases were collected, dried over anhydrous sodium sulfate, filtered, then concentrated under reduced pressure, and purified by silica gel column chromatography to yield the product.

After the product was obtained from protective group-protected formula (M), it was de-protected by hydrochloric acid or trifluoroacetic acid to yield the final product.

### Example 5: Preparation of Compound 2

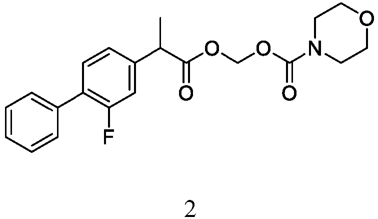

General Synthetic Method 2 was employed for preparation to yield a colorless liquid with a yield of 64.7%.

¹H NMR (400 MHz, DMSO-*d*₆) δ 7.54 (dt, *J* = 8.2, 1.6 Hz, 2H), 7.51 - 7.45 (m, 3H), 7.43 - 7.38 (m, 1H), 7.27 - 7.18 (m, 2H), 5.78 - 5.68 (m, 2H), 3.97 (q, *J* = 7.1 Hz, 1H), 3.48 (d, *J* = 14.3 Hz, 4H), 3.30 (s, 4H), 1.43 (d, *J* = 7.1 Hz, 3H).

### Example 6: Preparation of Compound 13

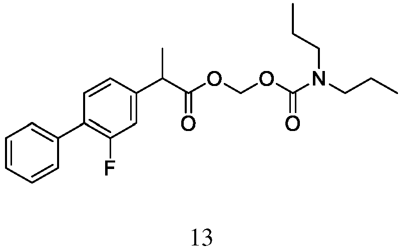

General Synthetic Method 2 was employed for preparation to yield a colorless liquid with a yield of 50.9%.

¹H NMR (400 MHz, DMSO-*d*₆) δ 7.56 - 7.44 (m, 5H), 7.43 - 7.37 (m, 1H), 7.25 - 7.18 (m, 2H), 5.74 (d, *J =* 5.9 Hz, 1H), 5.65 (s, 1H), 3.94 (q, *J =* 7.1 Hz, 1H), 3.13 - 2.99 (m, 4H), 1.42 (t, *J =* 6.5 Hz, 5H), 1.33 (q, *J =* 7.4 Hz, 2H), 0.73 (dt, *J =* 20.5, 7.4 Hz, 6H).

### Example 7: Preparation of Compound 14

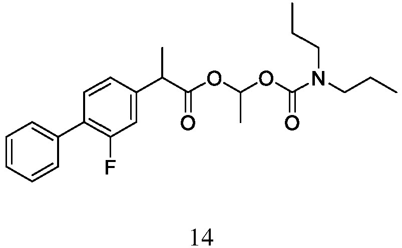

General Synthetic Method 2 was employed for preparation to yield a colorless liquid with a yield of 32.4%.

¹H NMR (400 MHz, DMSO-*d*₆) δ 7.57 - 7.43 (m, 5H), 7.43 - 7.38 (m, 1H), 7.27 - 7.14 (m, 2H), 6.71 (dt, *J* = 14.6, 5.4 Hz, 1H), 3.89 (qd, *J* = 7.0, 2.3 Hz, 1H), 3.17 - 2.89 (m, 4H), 1.45 (d, *J* = 19.0 Hz, 2H), 1.43 - 1.37 (m, 6H), 1.36 - 1.21 (m, 2H), 0.83 - 0.63 (m, 6H).

### Example 8: Preparation of Compound 15

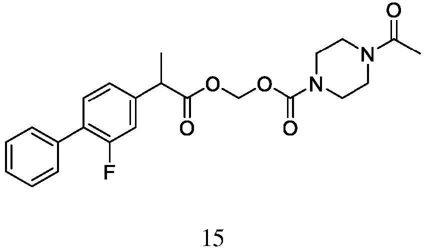

General Synthetic Method 2 was employed for preparation to yield a colorless liquid with a yield of 56.4%.

¹H NMR (400 MHz, DMSO-*d*₆) δ 7.54 - 7.44 (m, 5H), 7.43 - 7.37 (m, 1H), 7.28 - 7.19 (m, 2H), 5.78 - 5.68 (m, 2H), 3.97 (q, *J* = 7.1 Hz, 1H), 3.45 (s, 4H), 3.28 (s, 4H), 1.95 (s, 3H), 1.44 (d, *J* = 7.1 Hz, 3H).

### Example 9: Preparation of Compound 16

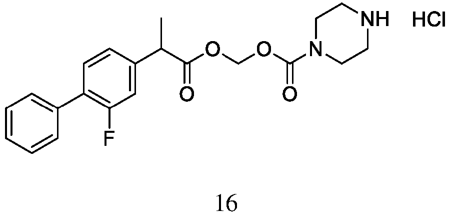

General Synthetic Method 2 was employed for preparation to yield a white solid with a yield of 44.6%.

¹H NMR (400 MHz, DMSO-*d*₆) δ 8.75 (s, 1H), 7.57 - 7.46 (m, 4H), 7.44 - 7.39 (m, 1H), 7.28 - 7.18 (m, 2H), 5.74 (s, 2H), 3.97 (q, *J* = 7.0 Hz, 1H), 3.54 (m, 4H), 3.09 (m, 4H), 1.44 (d, *J* = 7.1 Hz, 3H).

¹H NMR (400 MHz, DMSO-*d*₆) δ 8.75 (s, 2H), 7.56 - 7.38 (m, 6H), 7.26 - 7.20 (m, 2H), 5.75 (d, *J =* 8.0 Hz, 2H), 3.97 (q, *J =* 7.1 Hz, 1H), 3.58 - 3.51 (m, 4H), 3.09 (s, 4H), 1.44 (d, *J* = 7.1 Hz, 3H). (R.20.6, 21.11.2023)

### Example 10: Preparation of Compound 18

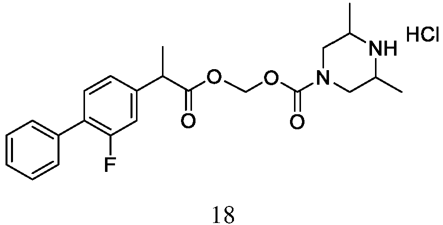

General Synthetic Method 2 was employed for preparation to yield a white solid with a yield of 36.4%.

¹H NMR (600 MHz, DMSO-*d*₆) δ 9.15 (d, *J =* 10.8 Hz, 1H), 8.52 (d, *J =* 11.1 Hz, 1H), 7.56 - 7.46 (m, 5H), 7.44 - 7.38 (m, 1H), 7.26 - 7.20 (m, 2H), 5.75 (d, *J =* 12.7 Hz, 2H), 4.07 (m, 1H), 3.97 (q, *J =* 7.1 Hz, 2H), 3.25 (s, 2H), 2.78 (m, 2H), 1.44 (d, *J* = 7.1 Hz, 3H), 1.19 (d, *J =* 6.4 Hz, 6H).

### Example 11: Preparation of Compound 20

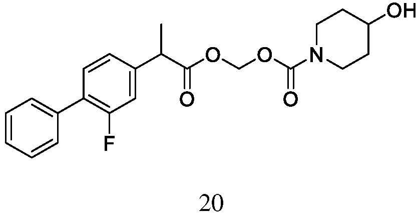

General Synthetic Method 2 was employed for preparation to yield a colorless oily product with a yield of 38.3%.

¹H NMR (400 MHz, DMSO-*d*₆) δ 7.56 - 7.45 (m, 5H), 7.43 - 7.38 (m, 1H), 7.27 - 7.18 (m, 2H), 5.75 - 5.65 (m, 2H), 4.76 (d, *J =* 3.9 Hz, 1H), 3.96 (q, *J* = 7.1 Hz, 1H), 3.61 (s, 3H), 3.09 - 2.99 (m, 2H), 1.70 - 1.57 (m, 2H), 1.43 (d, *J =* 7.1 Hz, 3H), 1.24 (s, 2H).

### Example 12: Preparation of Compound 21

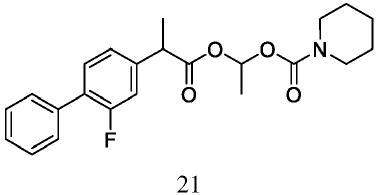

General Synthetic Method 2 was employed for preparation to yield a colorless oily product with a yield of 46.7%.

¹H NMR (400 MHz, DMSO-*d*₆) δ 7.57 - 7.43 (m, 5H), 7.39 (t, *J =* 7.2 Hz, 1H), 7.27 - 7.16 (m, 2H), 6.71 (dq, *J* = 12.9, 5.4 Hz, 1H), 3.90 (q, *J =* 7.1 Hz, 1H), 3.32 - 3.05 (m, 4H), 1.52 (q, *J =* 5.6 Hz, 1H), 1.48 - 1.29 (m, 11H).

### Example 13: Preparation of Compound 22

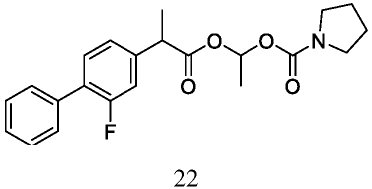

General Synthetic Method 2 was employed for preparation to yield a colorless oily product with a yield of 42.2%.

¹H NMR (400 MHz, DMSO-*d*₆) δ 7.60 - 7.44 (m, 5H), 7.43 - 7.37 (m, 1H), 7.28 - 7.15 (m, 2H), 6.71 (dq, *J =* 10.9, 5.4 Hz, 1H), 3.91 (q, *J =* 7.1 Hz, 1H), 3.25 (t, *J =* 5.2 Hz, 2H), 3.17 (q, *J =* 6.0 Hz, 2H), 1.85 - 1.66 (m, 4H), 1.49 - 1.32 (m, 6H).

### Example 14: Preparation of Compound 23

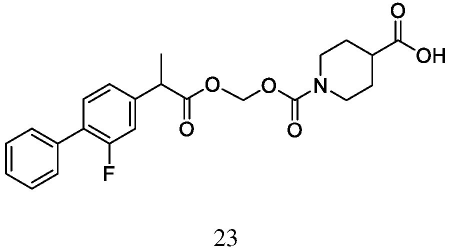

General Synthetic Method 2 was employed for preparation to yield a colorless liquid with a yield of 34.9%.

¹H NMR (600 MHz, DMSO-*d*₆) δ 12.29 (s, 1H), 7.55 - 7.45 (m, 5H), 7.40 (t, *J* = 7.3 Hz, 1H), 7.25 - 7.18 (m, 2H), 5.74 - 5.68 (m, 2H), 3.96 (q, *J =* 7.1 Hz, 1H), 3.80 (d, *J* = 40.6 Hz, 2H), 2.89 (s, 2H), 2.42 (s, 1H), 1.78 (d, *J =* 13.9 Hz, 2H), 1.43 (d, *J* = 7.1 Hz, 3H), 1.41 - 1.33 (m, 2H).

### Example 15: Preparation of Compound 24

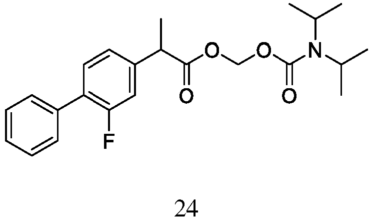

General Synthetic Method 2 was employed for preparation to yield a colorless liquid with a yield of 46.6%.

¹H NMR (400 MHz, DMSO-*d*₆) δ 7.54 - 7.45 (m, 5H), 7.41 (d, *J =* 6.8 Hz, 1H), 7.21 (d, *J =* 11.3 Hz, 2H), 5.75 (s, 1H), 5.67 (d, *J =* 5.9 Hz, 1H), 3.96 (d, *J =* 7.1 Hz, 2H), 3.68 (s, 1H), 1.43 (d, *J* = 7.1 Hz, 3H), 1.05 (m, 12H).

¹H NMR (400 MHz, DMSO-*d*₆) δ 7.54 - 7.45 (m, 5H), 7.43 - 7.38 (m, 1H), 7.25-7.18 (m, 2H), 5.75 (d, *J =* 5.9 Hz, 1H), 5.67 (d, *J* = 5.9 Hz, 1H), 3.96 (q, *J* = 7.1 Hz, 1H), 3.89 (s, 1H), 3.68 (s, 1H), 1.43 (d, *J* = 7.1 Hz, 3H), 1.05 (d, J = 18.9 Hz, 12H). (R.20.6, 21.11.2023)

### Example 16: Preparation of Compound 25

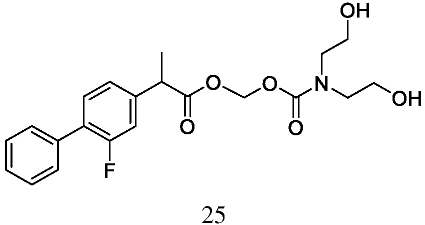

General Synthetic Method 2 was employed for preparation to yield a colorless liquid with a yield of 43.6%.

¹H NMR (400 MHz, DMSO-*d*₆) δ 7.56 - 7.45 (m, 5H), 7.43 - 7.38 (m, 1H), 7.26 - 7.19 (m, 2H), 5.72 - 5.67 (m, 2H), 4.75 (td, *J =* 5.4, 3.4 Hz, 2H), 3.96 (q, *J* = 7.1 Hz, 1H), 3.46 (q, *J =* 6.0 Hz, 2H), 3.39 (q, *J =* 6.0 Hz, 2H), 3.25 (m, 4H), 1.43 (d, *J* = 7.1 Hz, 3H).

### Example 17: Preparation of Compound 3

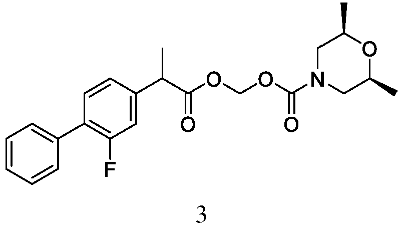

General Synthetic Method 2 was employed for preparation to yield a colorless liquid with a yield of 42.1%.

¹H NMR (400 MHz, DMSO-*d*₆) δ 7.55 - 7.51 (m, 2H), 7.51 - 7.45 (m, 3H), 7.44 - 7.38 (m, 1H), 7.28 - 7.18 (m, 2H), 5.75 (t, *J =* 4.8 Hz, 1H), 5.69 (d, *J =* 5.9 Hz, 1H), 3.96 (q, *J =* 7.1 Hz, 1H), 3.77 (d, *J =* 13.4 Hz, 1H), 3.64 (d, *J =* 13.1 Hz, 1H), 3.41 - 3.34 (m, 1H), 3.26 (s, 1H), 2.49 - 2.34 (m, 2H), 1.44 (d, *J* = 7.1 Hz, 3H), 1.02 (d, *J* = 6.6 Hz, 6H).

¹H NMR (400 MHz, DMSO-*d*₆) δ 7.55 - 7.51 (m, 2H), 7.51 - 7.45 (m, 3H), 7.44 - 7.38 (m, 1H), 7.25 - 7.21 (m, 2H), 5.74 (d, *J =* 4.8 Hz, 1H), 5.69 (d, *J* = 5.9 Hz, 1H), 3.96 (q, *J =* 7.1 Hz, 1H), 3.77 (d, *J =* 13.4 Hz, 1H), 3.64 (d, *J =* 13.1 Hz, 1H), 3.41 - 3.34 (m, 1H), 3.26 (s, 1H), 2.49 - 2.34 (m, 2H), 1.44 (d, *J* = 7.1 Hz, 3H), 1.02 (d, *J =* 6.6 Hz, 6H). (R.20.6, 21.11.2023)

### Example 18: Preparation of Compound 4

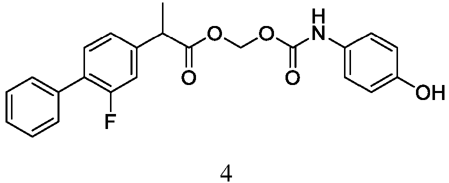

General Synthetic Method 2 was employed for preparation to yield a white solid with a yield of 45.2%.

¹H NMR (600 MHz, DMSO-*d*₆) δ 9.89 (s, 1H), 9.18 (s, 1H), 7.53 (d, *J =* 8.4 Hz, 2H), 7.47 (q, *J* = 8.1 Hz, 4H), 7.41 - 7.35 (m, 4H), 7.30 (d, *J =* 9.2 Hz, 2H), 6.67 (d, *J* = 8.9 Hz, 2H), 3.85 (q, *J =* 7.0 Hz, 1H), 1.44 (d, *J =* 6.9 Hz, 3H).

### Example 19: Preparation of Compound 5

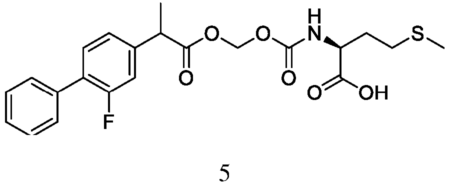

General Synthetic Method 2 was employed for preparation to yield a colorless liquid with a yield of 51.7%.

¹H NMR (400 MHz, DMSO) δ 7.55 (d, *J* = 7.6 Hz, 2H), 7.51 - 7.43 (m, 3H), 7.40 (t, *J* = 7.3 Hz, 1H), 7.30 - 7.18 (m, 2H), 6.77 (s, 1H), 5.65 (ddd, *J* = 15.7, 6.0, 3.4 Hz, 2H), 3.95 (q, *J* = 7.1 Hz, 1H), 3.62 (d, *J =* 5.2 Hz, 1H), 2.36 (dd, *J* = 12.9, 4.5 Hz, 2H), 1.98 (t, *J =* 9.6 Hz, 3H), 1.86 (s, 1H), 1.78 - 1.72 (m, 1H), 1.43 (dd, *J* = 7.1, 1.7 Hz, 3H).

¹H NMR (400 MHz, DMSO-*d*₆) δ 7.55 (d, *J =* 7.6 Hz, 2H), 7.51 - 7.43 (m, 3H), 7.40 (t, *J* = 7.3 Hz, 1H), 7.30 - 7.18 (m, 2H), 6.77 (s, 1H), 5.65 (ddd, *J* = 15.7, 6.0, 3.4 Hz, 2H), 3.95 (q, *J =* 7.1 Hz, 1H), 3.62 (d, *J =* 5.2 Hz, 1H), 2.36 (dd, *J* = 12.9, 4.5 Hz, 2H), 1.98 (t, *J =* 9.6 Hz, 3H), 1.86 (s, 1H), 1.78 - 1.72 (m, 1H), 1.43 (dd, *J =* 7.1, 1.7 Hz, 3H). (R.20.6, 21.11.2023)

### Example 20: Preparation of Compound 6

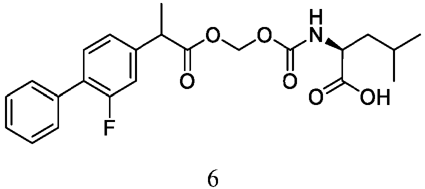

General Synthetic Method 2 was employed for preparation to yield a colorless liquid with a yield of 40.8%.

¹H NMR (400 MHz, DMSO-*d*₆) δ 12.61 (s, 1H), 7.89 (dd, *J =* 8.1, 3.8 Hz, 1H), 7.57 - 7.52 (m, 2H), 7.48 (td, *J =* 8.1, 2.2 Hz, 3H), 7.44 - 7.36 (m, 1H), 7.28 - 7.17 (m, 2H), 5.68 (qd, *J* = 6.1, 3.7 Hz, 2H), 3.94 (ddd, *J =* 13.0, 8.3, 3.3 Hz, 2H), 1.65 - 1.46 (m, 3H), 1.43 (d, *J* = 7.2 Hz, 3H), 0.89 - 0.76 (m, 6H).

### Example 21: Preparation of Compound 7

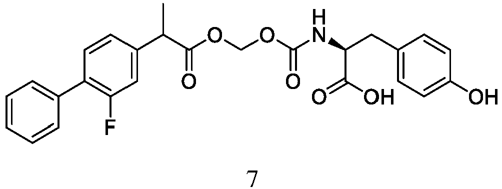

General Synthetic Method 2 was employed for preparation to yield a colorless liquid with a yield of 48.2%.

¹H NMR (400 MHz, DMSO-*d*₆) δ 12.75 (s, 1H), 9.22 (d, *J =* 3.1 Hz, 1H), 7.93 (dd, *J* = 8.3, 4.0 Hz, 1H), 7.54 (dd, *J* = 8.4, 1.5 Hz, 2H), 7.50 - 7.45 (m, 3H), 7.43 - 7.40 (m, 1H), 7.26 - 7.17 (m, 2H), 7.05 - 6.98 (m, 2H), 6.66 - 6.62 (m, 2H), 5.66 - 5.56 (m, 2H), 4.10 (q, *J =* 5.3 Hz, 2H), 4.08 - 4.03 (m, 1H), 3.92 (dd, *J* = 7.2, 4.8 Hz, 1H), 1.41 (dd, *J* = 7.1, 1.5 Hz, 3H).

### Example 22: Preparation of Compound 8

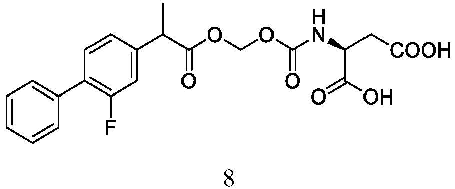

General Synthetic Method 2 was employed for preparation to yield a colorless liquid with a yield of 42.3%.

¹H NMR (400 MHz, DMSO-*d*₆) δ 7.95 (d, *J* = 8.3 Hz, 1H), 7.56 - 7.52 (m, 2H), 7.48 (t, *J* = 8.0 Hz, 3H), 7.43 - 7.37 (m, 1H), 7.28 - 7.19 (m, 2H), 5.72 - 5.64 (m, 2H), 4.32 (td, *J* = 8.1, 5.4 Hz, 1H), 3.96 (q, *J* = 7.1 Hz, 1H), 2.76 - 2.68 (m, 1H), 2.58 - 2.51 (m, 1H), 1.44 (d, *J =* 7.1 Hz, 3H).

### Example 23: Preparation of Compound 9

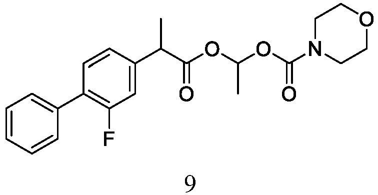

General Synthetic Method 2 was employed for preparation to yield a colorless oily liquid with a yield of 38.6%.

¹H NMR (400 MHz, DMSO-*d*₆) δ 7.57 - 7.45 (m, 5H), 7.44 - 7.37 (m, 1H), 7.29 - 7.20 (m, 2H), 6.71 (q, *J =* 5.4 Hz, 1H), 3.92 (q, *J =* 7.1 Hz, 1H), 3.54 (d, *J =* 11.3 Hz, 4H), 3.39 - 3.30 (m, 4H), 1.45 - 1.36 (m, 6H).

### Example 24: Preparation of Compound 26

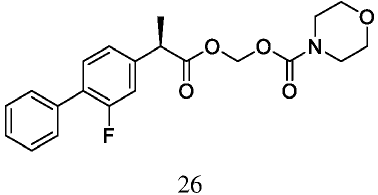

General Synthetic Method 2 was employed for preparation to yield a colorless liquid with a yield of 61.4%.

¹H NMR (600 MHz, DMSO) δ 7.50 (ddd, *J =* 15.3, 13.6, 7.9 Hz, 5H), 7.41 (t, *J* = 7.3 Hz, 1H), 7.26 - 7.19 (m, 2H), 5.73 (dd, *J* = 21.7, 6.7 Hz, 2H), 3.96 (q, *J =* 7.1 Hz, 1H), 3.50 (s, 4H), 2.51 (s, 4H), 1.44 (t, *J =* 8.1 Hz, 3H).

### Example 25: Preparation of Compound 27

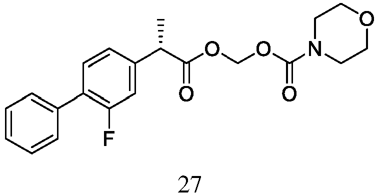

General Synthetic Method 2 was employed for preparation to yield a colorless liquid with a yield of 64.1%.

¹H NMR (600 MHz, DMSO) δ 7.50 (dd, *J =* 21.8, 13.5 Hz, 5H), 7.40 (t, *J =* 6.8 Hz, 1H), 7.21 (s, 2H), 5.74 (d, *J* = 6.5 Hz, 1H), 5.71 (d, *J* = 5.9 Hz, 1H), 3.97 (q, *J* = 7.1 Hz, 1H), 3.50 (s, 4H), 2.52 (s, 4H), 1.44 (d, *J =* 7.1 Hz, 3H).

### Example 26: Preparation of Compound 28

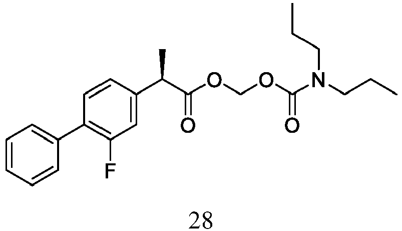

General Synthetic Method 2 was employed for preparation to yield a colorless liquid with a yield of 63.4%.

¹H NMR (400 MHz, DMSO) δ 7.53 - 7.46 (m, 5H), 7.42 - 7.38 (m, 1H), 7.21 (d, *J* = 9.4 Hz, 2H), 5.70 (dd, *J* = 33.6, 5.9 Hz, 2H), 3.94 (q, *J =* 7.1 Hz, 1H), 3.07 (ddd, *J* = 23.6, 14.1, 6.9 Hz, 4H), 1.46 - 1.23 (m, 7H), 0.79 - 0.63 (m, 6H).

### Example 27: Preparation of Compound 29

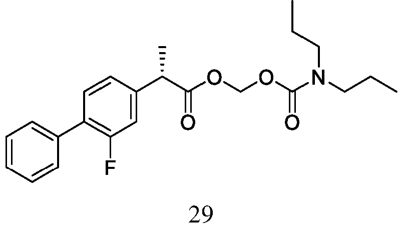

General Synthetic Method 2 was employed for preparation to yield a colorless liquid with a yield of 62.5%.

¹H NMR (400 MHz, DMSO) δ 7.56 - 7.37 (m, 6H), 7.21 (d, *J* = 9.4 Hz, 2H), 5.70 (dd, *J* = 33.7, 5.9 Hz, 2H), 3.94 (q, *J =* 7.1 Hz, 1H), 3.12 - 2.99 (m, 4H), 1.43 (d, *J =* 7.1 Hz, 3H), 1.41 - 1.22 (m, 4H), 0.73 (dt, *J =* 20.2, 7.4 Hz, 6H).

### Example 28: Preparation of Compound 30

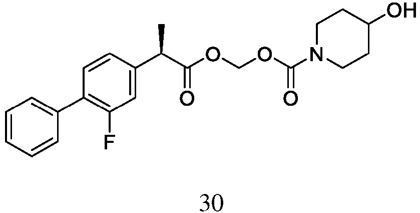

General Synthetic Method 2 was employed for preparation to yield a colorless liquid with a yield of 67.6%.

¹H NMR (600 MHz, DMSO) δ 7.56 - 7.37 (m, 6H), 7.21 (t, *J* = 8.2 Hz, 2H), 5.70 (dd, *J* = 20.6, 5.9 Hz, 2H), 4.72 (d, *J* = 3.7 Hz, 1H), 3.96 (q, *J* = 7.1 Hz, 1H), 3.62 (s, 3H), 3.05 (s, 2H), 1.67 - 1.61 (m, 2H), 1.43 (d, *J =* 7.1 Hz, 2H), 1.25 (s, 3H).

### Example 29: Preparation of Compound 31

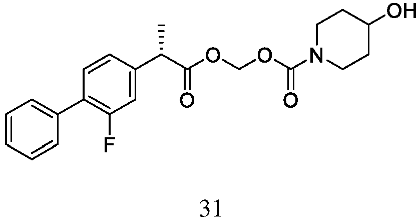

General Synthetic Method 2 was employed for preparation to yield a colorless liquid with a yield of 59.4%.

¹H NMR (600 MHz, DMSO) δ 7.59 - 7.37 (m, 6H), 7.22 (t, *J* = 8.2 Hz, 2H), 5.70 (dd, *J* = 20.6, 5.9 Hz, 2H), 4.72 (d, *J* = 3.7 Hz, 1H), 3.96 (q, *J* = 7.1 Hz, 1H), 3.62 (s, 3H), 3.05 (s, 2H), 1.67 - 1.61 (m, 2H), 1.43 (d, *J =* 7.1 Hz, 3H), 1.24 (s, 2H).

### General Synthetic Method 3:

Method D was adopted for synthesis.

Formula (A) (1.0 eq.) was dissolved into anhydrous DCM, and oxalyl chloride (3.0 eq.) or acetyl chloride (1.2 to 1.5 eq.) and DMF at a catalytic amount were added dropwise, and stirred at room temperature for 0.5 h. The solution was drained to yield formula (G). Formula (G) was dissolved into a mixed solution of ethyl acetate:water (2:1) or into a DCM solution, added dropwise to a mixed system of formula (F) (2.0 eq.) and potassium carbonate (6.0 eq.) or triethylamine (1.5 eq.), and stirred at -20°C to 25°C for 4 to 12 h. 10 mL of ethyl acetate was added. The organic phases were washed with water (10 mL). The organic phases were collected, dried over anhydrous sodium sulfate, filtered, concentrated under reduced pressure, and purified by column chromatography to yield the product.

### Example 30: Preparation of Compound 32

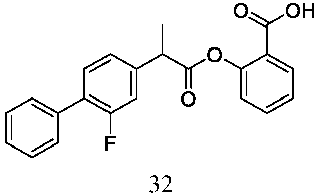

General Synthetic Method 3 was employed for preparation to yield a white solid with a yield of 42.7%.

¹H NMR (400 MHz, DMSO-*d*₆) δ 13.20 (s, 1H), 7.94 (dd, *J =* 7.8, 1.7 Hz, 1H), 7.63 (td, *J =* 7.7, 1.8 Hz, 1H), 7.60 - 7.53 (m, 3H), 7.52 - 7.46 (m, 2H), 7.44 - 7.33 (m, 4H), 7.14 (dd, *J* = 8.1, 1.2 Hz, 1H), 4.19 (q, *J =* 7.2 Hz, 1H), 1.59 (d, *J =* 7.2 Hz, 3H).

### Example 31: Preparation of Compound 33

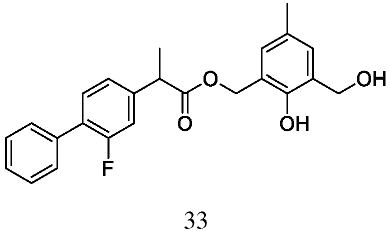

General Synthetic Method 3 was employed for preparation to yield a colorless oily product with a yield of 56.5%.

¹H NMR (400 MHz, DMSO-*d*₆) δ 8.55 (s, 1H), 7.56 - 7.52 (m, 2H), 7.48 (t, *J* = 7.6 Hz, 3H), 7.43 - 7.38 (m, 1H), 7.27 (t, *J =* 8.0 Hz, 2H), 7.00 (s, 1H), 6.73 (s, 1H), 5.31 (t, *J* = 5.3 Hz, 1H), 5.15 - 5.05 (m, 2H), 4.53 (d, *J =* 4.0 Hz, 2H), 3.95 (q, *J =* 7.1 Hz, 1H), 2.12 (s, 3H), 1.46 (d, *J* = 7.1 Hz, 3H).

### General Synthetic Method 4:

Method E was adopted for synthesis.

### Step 1:

Formula (J) (1.0 eq.), sodium bicarbonate (3.8 eq.), and tetrabutylammonium hydrogen sulfate (0.1 eq.) were dissolved into a mixed solution of dichloromethane:water (1:1), and stirred at room temperature for 5 min. Chloromethyl chlorosulfonate (1.1 to 2 eq.) was dissolved into DCM, added dropwise to the above reaction solution, and stirred at room temperature for 1 h. The organic phases were washed with water (20 mL), dried over anhydrous sodium sulfate, filtered, and concentrated under reduced pressure to yield formula (H).

Alternatively, formula (J) (1 eq.) was dissolved into DMF. To the solution were added imidazole (2 eq.) and tert-butyldimethylsilyl chloride (2 eq.) or benzyl bromide (2 eq.) in sequence, and stirred at room temperature for 1 h. The mixture was poured into 15 mL of water, and extracted with ethyl acetate (10 mL*3). The organic phases were washed with a saturated aqueous sodium chloride solution (20 mL) three times. The organic phases were collected, dried over anhydrous sodium sulfate, and distilled under reduced pressure to yield protective group-protected formula (J). Subsequently, the first step reaction was carried out to yield protective group-protected formula (H).

Alternatively, formula (J) (1 eq.) was dissolved into dry DCM. Oxalyl chloride (1.5 eq.) was added under nitrogen protection, and then one drop of DMF was added, stirred at room temperature for 1 h, and distilled under reduced pressure to yield an acyl chloride compound. The acyl chloride compound was dissolved into DCM, and zinc chloride (0.02 eq.) was added. Subsequently, acetaldehyde (1 eq.) was added at -15°C under nitrogen protection. The reaction solution was reacted at room temperature for 16 h, and concentrated under reduced pressure. Afterwards, the residue was dissolved into 20 mL of ethyl acetate. The resultant was washed with water (20mL), a saturated sodium bicarbonate solution (20 mL), and a saturated aqueous sodium chloride solution (20 mL) in sequence. The organic phases were collected, dried over anhydrous sodium sulfate, and filtered to obtain a filtrate. The filtrate was evaporated to dryness under reduced pressure to yield a compound of formula (H).

### Step 2:

Formula (A) (1.0 eq.) was dissolved into DMF. Triethylamine (1.5 eq.) or potassium carbonate (1.6 to 2.0 eq.) was added, and sodium iodide (1.1 eq.) might or might not be added, and stirred at 20°C to 50°C. Formula (H) or protective group-protected formula (H) (1.2 eq.) was dissolved into DMF, slowly added to the above reaction solution, and reacted for 3 to 4 h. 20 mL of ethyl acetate was added. Thereafter, the organic phases were washed with water (20 mL) and a saturated aqueous sodium chloride solution (20 mL) in sequence. The organic phases were collected, dried over anhydrous sodium sulfate, filtered, concentrated under reduced pressure, and purified by silica gel column chromatography to yield the product formula (III) or protective group-containing formula (III).

The tert-butyldimethylsilyl was de-protected from the protective group-protected formula (III) by hydrogen reduction or by acid or base or tetrabutylammonium fluoride to yield formula (III).

### Example 32: Preparation of Compound 34

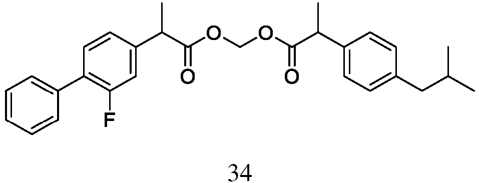

General Synthetic Method 4 was employed for preparation to yield a colorless oily product with a yield of 20.0%.

¹H NMR (400 MHz, DMSO-*d*₆) δ 7.56 - 7.44 (m, 5H), 7.41 (td, *J =* 7.0, 1.6 Hz, 1H), 7.23 - 7.12 (m, 2H), 7.12 - 7.01 (m, 4H), 5.81 - 5.61 (m, 2H), 3.90 (p, *J* = 7.1 Hz, 1H), 3.76 (p, *J =* 6.9 Hz, 1H), 2.36 (t, *J =* 7.2 Hz, 2H), 1.75 (dh, *J* = 13.2, 6.6 Hz, 1H), 1.34 (ddd, *J* = 19.4, 11.2, 7.2 Hz, 6H), 0.81 (dd, *J* = 6.6, 4.4 Hz, 6H).

### Example 33: Preparation of Compound 35

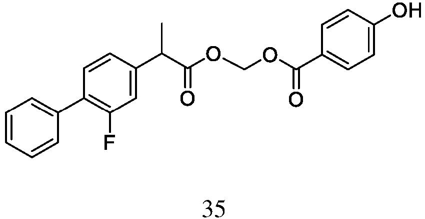

General Synthetic Method 4 was employed for preparation to yield a colorless oily product with a yield of 38.7%.

¹H NMR (600 MHz, DMSO-*d*₆) δ 10.48 (s, 1H), 7.80 - 7.75 (m, 2H), 7.46 (d, *J* = 4.6 Hz, 4H), 7.43 - 7.37 (m, 2H), 7.25 - 7.18 (m, 2H), 6.88 - 6.82 (m, 2H), 5.96 - 5.89 (m, 2H), 3.99 (q, *J* = 7.1 Hz, 1H), 1.44 (d, *J* = 7.1 Hz, 3H).

### Example 34: Preparation of Compound 36

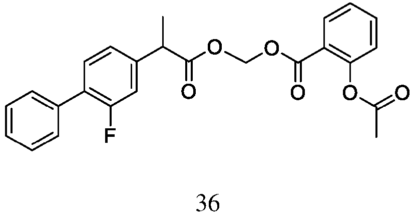

General Synthetic Method 4 was employed for preparation to yield a white solid with a yield of 47.4%.

¹H NMR (600 MHz, DMSO-*d*₆) δ 7.88 (dd, *J =* 7.8, 1.7 Hz, 1H), 7.75 - 7.69 (m, 1H), 7.51 - 7.37 (m, 7H), 7.28 - 7.20 (m, 3H), 5.96 - 5.90 (m, 2H), 4.01 (q, *J* = 7.1 Hz, 1H), 2.24 (s, 3H), 1.46 (d, *J* = 7.1 Hz, 3H).

### Example 35: Preparation of Compound 37

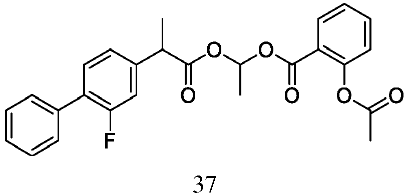

General Synthetic Method 4 was employed for preparation to yield a colorless oily product with a yield of 47.6%.

¹H NMR (400 MHz, DMSO-*d*₆) δ 7.88 (dd, *J =* 7.8, 1.7 Hz, 1H), 7.75 - 7.69 (m, 1H), 7.49 - 7.38 (m, 7H), 7.28 - 7.20 (m, 3H), 5.95 - 5.91 (m, 1H), 4.02 (dd, *J* = 7.1, 5.8 Hz, 1H), 2.24 (s, 3H), 1.46 (d, *J =* 7.1 Hz, 3H).

### Example 36: Preparation of Compound 38

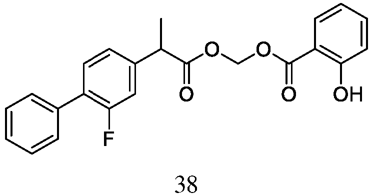

General Synthetic Method 4 was employed for preparation to yield a colorless oily product with a yield of 43.4%.

¹H NMR (600 MHz, DMSO-*d*₆) δ 10.29 (s, 1H), 7.67 (dd, *J* = 7.9, 1.8 Hz, 1H), 7.55 - 7.38 (m, 7H), 7.27 - 7.20 (m, 2H), 6.99 (d, *J =* 8.4 Hz, 1H), 6.91 (t, *J* = 7.6 Hz, 1H), 6.04 - 5.95 (m, 2H), 4.02 (q, *J* = 7.1 Hz, 1H), 1.46 (d, *J* = 7.1 Hz, 3H).

### Example 37: Preparation of Compound 39

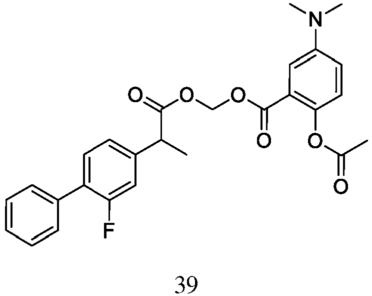

General Synthetic Method 4 was employed for preparation to yield a colorless oily product with a yield of 59.2%.

¹H NMR (400 MHz, DMSO-*d*₆) δ 7.51 - 7.37 (m, 6H), 7.30 - 7.15 (m, 2H), 7.09 - 6.95 (m, 3H), 5.91 (q, *J =* 6.0 Hz, 2H), 4.00 (q, *J =* 7.1 Hz, 1H), 2.88 (s, 6H), 2.18 (s, 3H), 1.45 (d, *J* = 7.1 Hz, 3H).

### Example 38: Preparation of Compound 40

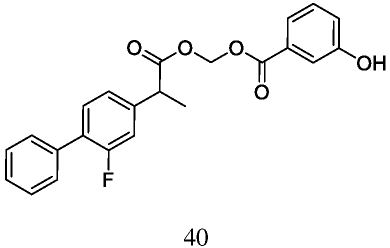

General Synthetic Method 4 was employed for preparation to yield a colorless oily product with a yield of 61.4%.

¹H NMR (400 MHz, DMSO-*d*₆) δ 9.90 (s, 1H), 7.48 - 7.38 (m, 6H), 7.37 - 7.30 (m, 3H), 7.26 - 7.18 (m, 2H), 7.06 (ddd, *J* = 7.4, 2.7, 1.7 Hz, 1H), 5.95 (q, *J =* 5.9 Hz, 2H), 4.01 (q, *J =* 7.1 Hz, 1H), 1.45 (d, *J =* 7.1 Hz, 3H).

### Example 39: Preparation of Compound 41

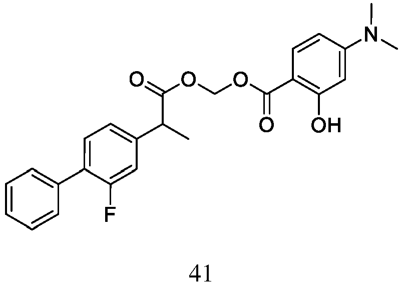

General Synthetic Method 4 was employed for preparation to yield a colorless oily product with a yield of 46.7%.

¹H NMR (400 MHz, DMSO-*d*₆) δ 10.35 (s, 1H), 7.46 (d, *J =* 5.4 Hz, 4H), 7.44 - 7.36 (m, 3H), 7.22 (dd, *J* = 11.9, 1.7 Hz, 1H), 7.19 (dd, *J* = 7.9, 1.8 Hz, 1H), 6.28 (dd, *J* = 9.2, 2.5 Hz, 1H), 6.07 (d, *J =* 2.5 Hz, 1H), 5.97 - 5.89 (m, 2H), 3.99 (t, *J =* 7.1 Hz, 1H), 2.97 (s, 6H), 1.44 (d, *J =* 7.1 Hz, 3H).

### Example 40: Preparation of Compound 42

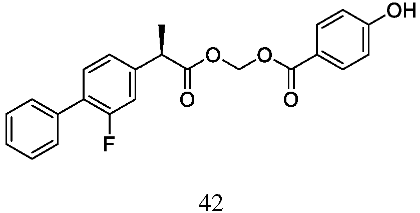

General Synthetic Method 4 was employed for preparation to yield a colorless oily product with a yield of 56.35%.

¹H NMR (600 MHz, DMSO) δ 10.48 (s, 1H), 7.78 (d, *J =* 8.6 Hz, 2H), 7.45 (t, *J* = 11.3 Hz, 4H), 7.42 - 7.36 (m, 2H), 7.21 (t, *J =* 11.3 Hz, 2H), 6.85 (d, *J =* 8.6 Hz, 2H), 5.92 (dd, *J* = 16.2, 5.9 Hz, 2H), 3.99 (q, *J =* 7.1 Hz, 1H), 1.44 (d, *J =* 7.1 Hz, 3H).

### Example 41: Preparation of Compound 43

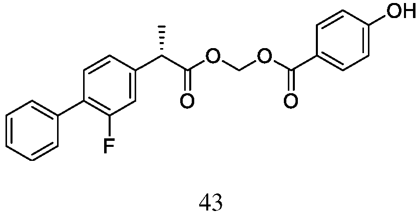

General Synthetic Method 4 was employed for preparation to yield a colorless oily product with a yield of 58.6%.

¹H NMR (600 MHz, DMSO) δ 10.48 (s, 1H), 7.78 (d, *J =* 8.6 Hz, 2H), 7.45 (t, *J* = 11.3 Hz, 4H), 7.42 - 7.36 (m, 2H), 7.21 (t, *J =* 11.3 Hz, 2H), 6.85 (d, *J =* 8.6 Hz, 2H), 5.92 (dd, *J* = 16.2, 5.9 Hz, 2H), 3.99 (q, *J =* 7.1 Hz, 1H), 1.44 (d, *J =* 7.1 Hz, 3H).

### Example 42: Preparation of Compound 44

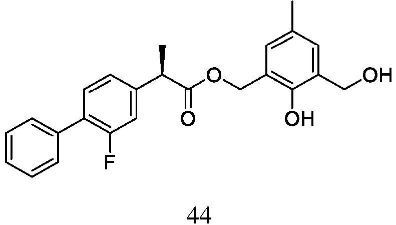

General Synthetic Method 3 was employed for preparation to yield a colorless oily product with a yield of 62.5%.

¹H NMR (600 MHz, DMSO) δ 8.51 (s, 1H), 7.56 - 7.43 (m, 6H), 7.40 (t, *J =* 7.3 Hz, 1H), 7.26 (t, *J =* 8.4 Hz, 2H), 6.99 (s, 1H), 6.72 (s, 1H), 5.27 (t, *J =* 5.3 Hz, 1H), 5.09 (q, *J =* 12.7 Hz, 2H), 4.52 (d, *J =* 4.8 Hz, 2H), 2.11 (s, 3H), 1.45 (d, *J =* 7.1 Hz, 3H).

### Example 43: Preparation of Compound 45

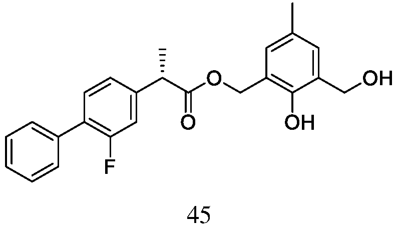

General Synthetic Method 3 was employed for preparation to yield a colorless oily product with a yield of 69.4%.

¹H NMR (600 MHz, DMSO) δ 8.52 (s, 1H), 7.57 - 7.45 (m, 5H), 7.40 (t, *J* = 7.4 Hz, 1H), 7.26 (t, *J =* 8.4 Hz, 2H), 6.99 (s, 1H), 6.72 (s, 1H), 5.28 (t, *J =* 5.3 Hz, 1H), 5.09 (q, *J =* 12.7 Hz, 2H), 4.52 (d, *J =* 5.1 Hz, 2H), 3.95 (q, *J =* 7.0 Hz, 1H), 2.11 (s, 3H), 1.45 (d, *J* = 7.1 Hz, 3H).

### General Synthetic Method 5:

Method C was adopted for synthesis.

Formula (A) (1.0 eq.), formula (F) (1.2 eq.), EDCI (1.5 eq.), and DMAP (0.1 eq.) were added to 5 mL of DMF, and stirred at room temperature for 12 h. 50 mL of ethyl acetate was added. Subsequently, the organic phases were washed with a saturated aqueous ammonium chloride solution (50 mL*2), water (50 mL), and a saturated aqueous sodium chloride solution (50 mL) in sequence. The organic phases were collected, dried over anhydrous sodium sulfate, filtered, concentrated under reduced pressure, and purified by column chromatography to yield the product.

### Example 44: Preparation of Compound 46

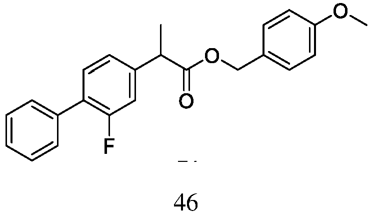

General Synthetic Method 5 was employed for preparation to yield a colorless oily product with a yield of 51.7%.

¹H NMR (400 MHz, DMSO-*d*₆) δ 7.57 - 7.44 (m, 5H), 7.44 - 7.37 (m, 1H), 7.27 - 7.18 (m, 4H), 6.95 - 6.85 (m, 2H), 5.11 - 5.00 (m, 2H), 3.92 (q, *J =* 7.1 Hz, 1H), 3.73 (s, 3H), 1.43 (d, *J =* 7.1 Hz, 3H).

### Example 45: Preparation of Compound 47

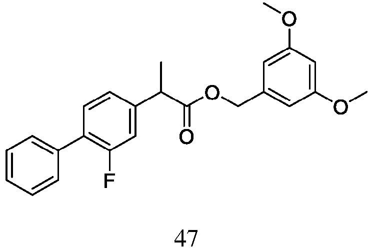

General Synthetic Method 4 was employed for preparation to yield a white solid with a yield of 92.9 %.

¹H NMR (600 MHz, DMSO-*d*₆) δ 7.59 - 7.45 (m, 5H), 7.40 (t, *J =* 7.3 Hz, 1H), 7.26 (t, *J =* 10.2 Hz, 2H), 6.40 (m, 3H), 5.07 (d, *J* = 22.8 Hz, 2H), 3.99 (d, *J* = 7.1 Hz, 1H), 3.67 (s, 6H), 1.47 (d, *J =* 7.1 Hz, 3H).

### Example 46: Preparation of Compound 48

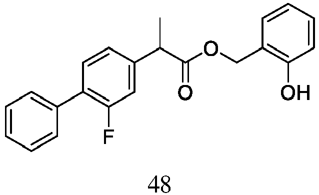

General Synthetic Method 3 was employed for preparation to yield a white solid with a yield of 40.7%.

¹H NMR (400 MHz, DMSO-*d*₆) δ 9.73 (s, 1H), 7.57 - 7.38 (m, 6H), 7.27 - 7.19 (m, 2H), 7.13 (m, 2H), 6.83 (dd, *J* = 8.1, 1.1 Hz, 1H), 6.75 (td, *J* = 7.5, 1.2 Hz, 1H), 5.08 (d, *J* = 3.8 Hz, 2H), 3.94 (q, *J* = 7.1 Hz, 1H), 1.45 (d, *J* = 7.1 Hz, 3H).

### Example 47: Preparation of Compound 49

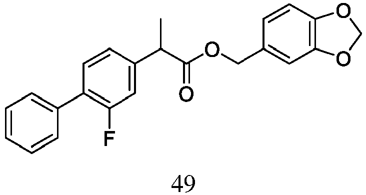

General Synthetic Method 3 was employed for preparation to yield a colorless liquid with a yield of 77.5%.

¹H NMR (600 MHz, CDCl₃) δ 7.53 (d, *J =* 7.8 Hz, 2H), 7.43 (t, *J =* 7.6 Hz, 2H), 7.36 (m, 2H), 7.15 - 7.07 (m, 2H), 6.80 - 6.70 (m, 3H), 5.93 (m, 2H), 5.08 - 4.93 (m, 2H), 3.77 (q, *J* = 7.2 Hz, 1H), 1.53 (d, *J =* 7.1 Hz, 3H).

### Example 48: Preparation of Compound 50

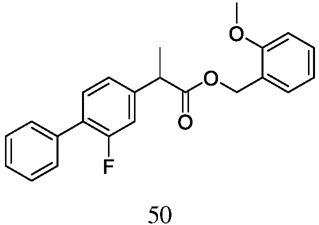

General Synthetic Method 5 was employed for preparation to yield a colorless liquid with a yield of 60.3%.

¹H NMR (600 MHz, DMSO-*d*₆) δ 7.58 - 7.52 (m, 2H), 7.48 (m, 3H), 7.45 - 7.37 (m, 1H), 7.31 (td, *J =* 7.9, 1.8 Hz, 1H), 7.27 - 7.14 (m, 3H), 7.00 (d, *J =* 8.2 Hz, 1H), 6.90 (t, *J =* 7.4 Hz, 1H), 5.54 - 4.86 (m, 2H), 3.94 (q, *J =* 7.1 Hz, 1H), 3.74 (s, 3H), 1.45 (d, *J* = 7.1 Hz, 3H).

### Example 49: Preparation of Compound 51

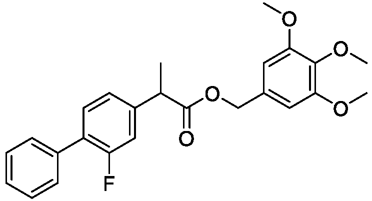

General Synthetic Method 3 was employed for preparation to yield a colorless liquid with a yield of 48.3%.

¹H NMR (400 MHz, DMSO-*d*₆) δ 7.55 - 7.45 (m, 5H), 7.45 - 7.37 (m, 1H), 7.30 - 7.24 (m, 2H), 6.55 (s, 2H), 5.14 - 5.00 (m, 2H), 3.99 (q, *J* = 7.1 Hz, 1H), 3.68 (s, 6H), 3.62 (s, 3H), 1.47 (d, *J* = 7.2 Hz, 3H).

### Example 50: Preparation of Compound 52

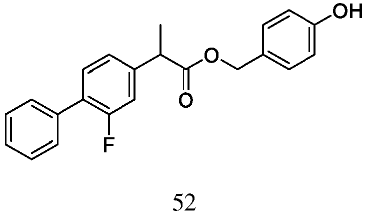

General Synthetic Method 5 was employed for preparation to yield a colorless liquid with a yield of 5.6%.

¹H NMR (600 MHz, DMSO-*d*₆) δ 9.46 (s, 1H), 7.63 - 7.32 (m, 6H), 7.24 - 7.05 (m, 4H), 6.71 (d, *J =* 8.4 Hz, 2H), 5.00 (q, *J =* 12.0 Hz, 2H), 3.91 (q, *J =* 7.1 Hz, 1H), 1.43 (d, *J* = 7.1 Hz, 3H).

### General Synthetic Method 6:

Method A was adopted for synthesis.

### Step 1: (synthesized by step 1)

Acid (1 eq.) was dispersed in DCM. An aqueous solution of tetrabutylammonium hydrogen sulfate and sodium bicarbonate (4 eq.) as catalysts was added. Chloromethyl chlorosulfonate (1.2 eq.) was added while stirring. The reaction solution was reacted at room temperature for 4 h and left stand for layering. The organic phases were collected, dried over anhydrous sodium sulfate, concentrated under reduced pressure, and purified by silica gel column chromatography to yield chlorinated formula (B).

### Step 2: (synthesized by step 2)

Chlorinated formula (B) was dissolved into acetonitrile, and sodium iodide (1.2 eq.) was added and reacted at 40°C for 1 h to yield iodinated formula (B).

### Step 3:

To the iodinated formula (B), formula (A) (1 eq.) and potassium carbonate (1.2 eq.) were added and further reacted for 10 h. The solvent was removed under reduced pressure, and ethyl acetate and saturated sodium chloride were added for extraction. The organic phases were collected, dried over anhydrous sodium sulfate, concentrated and separated to yield the product formula (II).

### Example 51: Preparation of Compound 53

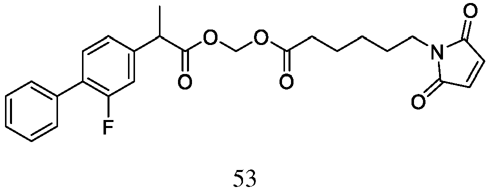

General Synthetic Method 6 was employed for preparation to yield a colorless liquid with a yield of 65.32%.

¹H NMR (400 MHz, Chloroform-d) δ 7.53 (dt, *J =* 8.0, 1.5 Hz, 2H), 7.48 - 7.34 (m, 4H), 7.17 - 7.08 (m, 2H), 6.67 (s, 2H), 5.75 (q, *J =* 5.6 Hz, 2H), 3.80 (q, *J =* 7.2 Hz, 1H), 3.47 (t, *J =* 7.2 Hz, 2H), 2.30 (t, *J =* 7.5 Hz, 2H), 1.67 - 1.50 (m, 8H), 1.34 - 1.23 (m, 3H).

¹H NMR (400 MHz, Chloroform-*d*) δ 7.53 (dt, *J =* 8.0, 1.5 Hz, 2H), 7.48 - 7.34 (m, 4H), 7.17 - 7.08 (m, 2H), 6.67 (s, 2H), 5.75 (q, *J =* 5.6 Hz, 2H), 3.80 (q, *J =* 7.2 Hz, 1H), 3.47 (t, *J =* 7.2 Hz, 2H), 2.30 (t, *J =* 7.5 Hz, 2H), 1.67 - 1.50 (m, 7H), 1.34 - 1.23 (m, 2H). (R.20.6, 21.11.2023)

### Example 52: Preparation of Compound 54

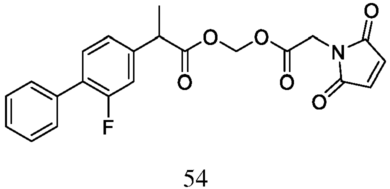

General Synthetic Method 6 was employed for preparation to yield a colorless liquid with a yield of 12.33%.

¹H NMR (400 MHz, Chloroform-*d*) δ 7.55 (d, *J =* 7.6 Hz, 2H), 7.48 - 7.36 (m, 4H), 7.16 - 7.09 (m, 2H), 5.84 - 5.76 (m, 2H), 5.74 (s, 2H), 3.81 (q, *J =* 7.1 Hz, 1H), 2.80 (s, 3H), 1.58 (s, 2H).

### Example 53: Preparation of Compound 55

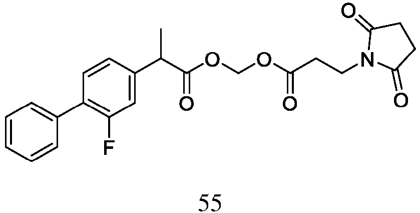

General Synthetic Method 6 was employed for preparation to yield a colorless liquid with a yield of 19.87%.

¹H NMR (400 MHz, Chloroform-*d*) δ 7.54 (dt, J = 6.8, 1.5 Hz, 2H), 7.47 - 7.34 (m, 4H), 7.17 - 7.09 (m, 2H), 5.77 - 5.70 (m, 2H), 3.88 - 3.77 (m, 4H), 2.76 - 2.71 (m, 3H), 2.65 (td, J = 7.0, 0.9 Hz, 2H), 1.57 (s, 3H).

### Example 54: Preparation of Compound 56

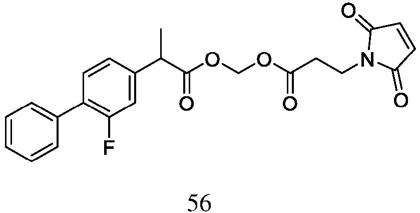

General Synthetic Method 6 was employed for preparation to yield a colorless liquid with a yield of 21.66%.

¹H NMR (400 MHz, Chloroform-*d*) δ 7.56 - 7.52 (m, 2H), 7.48 - 7.34 (m, 4H), 7.17 - 7.09 (m, 2H), 6.68 (s, 2H), 5.79 - 5.70 (m, 2H), 3.81 (td, *J =* 7.1, 3.5 Hz, 3H), 2.69 - 2.64 (m, 2H), 1.56 (d, *J =* 7.2 Hz, 3H).

### Example 55: Preparation of Compound 57

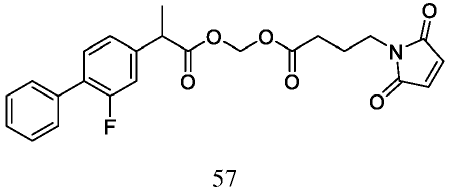

General Synthetic Method 6 was employed for preparation to yield a colorless liquid with a yield of 35.37%.

¹H NMR (400 MHz, Chloroform-*d*) δ 7.53 (dt, *J =* 8.2, 1.5 Hz, 2H), 7.50 - 7.33 (m, 4H), 7.19 - 7.06 (m, 2H), 6.66 (s, 2H), 5.84 - 5.70 (m, 2H), 3.81 (q, *J =* 7.1 Hz, 1H), 3.55 (t, *J =* 6.8 Hz, 2H), 2.33 (d, *J =* 7.4 Hz, 2H), 1.89 (p, *J =* 7.1 Hz, 2H), 1.56 (d, *J* = 7.2 Hz, 3H).

### Example 56: Preparation of Compound 58

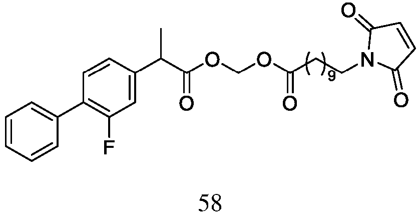

General Synthetic Method 6 was employed for preparation to yield a colorless liquid with a yield of 55.78%.

¹H NMR (400 MHz, Chloroform-*d*) δ 7.53 (dt, *J =* 8.1, 1.5 Hz, 2H), 7.48 - 7.32 (m, 4H), 7.18 - 7.07 (m, 2H), 6.68 (s, 2H), 5.80 - 5.72 (m, 2H), 3.79 (q, *J =* 7.2 Hz, 1H), 3.54 - 3.46 (m, 2H), 2.34 - 2.26 (m, 2H), 1.62 - 1.50 (m, 7H), 1.24 (dt, *J* = 11.6, 3.6 Hz, 12H).

### Example 57: Preparation of Compound 59

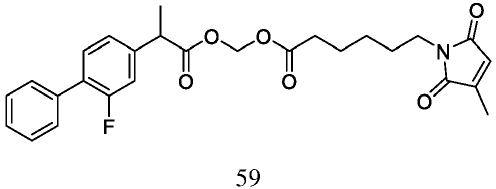

General Synthetic Method 6 was employed for preparation to yield a colorless liquid with a yield of 56.65%.

¹H NMR (400 MHz, Chloroform-*d*) δ 7.53 (dt, *J =* 8.0, 1.5 Hz, 2H), 7.48 - 7.32 (m, 4H), 7.15 (d, *J =* 1.8 Hz, 1H), 7.14 - 7.07 (m, 1H), 6.29 (q, *J =* 1.8 Hz, 1H), 5.75 (q, *J* = 5.6 Hz, 2H), 3.80 (q, *J =* 7.1 Hz, 1H), 3.45 (t, *J =* 7.2 Hz, 2H), 2.30 (t, *J* = 7.5 Hz, 2H), 2.07 (d, *J =* 1.9 Hz, 3H), 1.65 - 1.49 (m, 6H), 1.34 - 1.21 (m, 3H).

### Example 58: Preparation of Compound 60

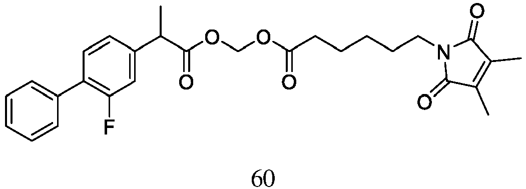

General Synthetic Method 6 was employed for preparation to yield a colorless liquid with a yield of 54.53%.

¹H NMR (400 MHz, Chloroform-*d*) δ 7.53 (dt, *J =* 8.1, 1.5 Hz, 2H), 7.48 - 7.32 (m, 4H), 7.15 (d, *J =* 1.8 Hz, 1H), 7.14 - 7.07 (m, 1H), 5.80 - 5.71 (m, 2H), 3.80 (q, *J* = 7.2 Hz, 1H), 3.44 (t, *J =* 7.2 Hz, 2H), 2.30 (t, *J =* 7.5 Hz, 2H), 1.95 (s, 6H), 1.64 - 1.45 (m, 6H), 1.28 (dtd, *J =* 10.7, 7.6, 7.2, 4.8 Hz, 3H).

### Example 59: Preparation of Compound 61

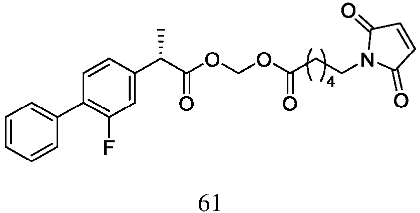

General Synthetic Method 6 was employed for preparation to yield a colorless liquid with a yield of 89.25%.

¹H NMR (400 MHz, Chloroform-d) δ 7.53 (dt, *J =* 8.2, 1.5 Hz, 2H), 7.49 - 7.32 (m, 4H), 7.17 - 7.07 (m, 2H), 6.67 (s, 2H), 5.75 (q, *J =* 5.6 Hz, 2H), 3.80 (q, *J =* 7.1 Hz, 1H), 3.48 (t, *J =* 7.2 Hz, 2H), 2.30 (t, *J* = 7.5 Hz, 2H), 1.67 - 1.50 (m, 8H), 1.32 - 1.21 (m, 3H).

¹H NMR (400 MHz, Chloroform-d) δ 7.53 (dt, *J =* 8.2, 1.5 Hz, 2H), 7.49 - 7.32 (m, 4H), 7.17 - 7.07 (m, 2H), 6.67 (s, 2H), 5.75 (q, *J =* 5.6 Hz, 2H), 3.80 (q, *J =* 7.1 Hz, 1H), 3.48 (t, *J =* 7.2 Hz, 2H), 2.30 (t, *J* = 7.5 Hz, 2H), 1.67 - 1.50 (m, 7H), 1.32 - 1.21 (m, 2H). (R.20.6, 21.11.2023)

### Example 60: Preparation of Compound 62

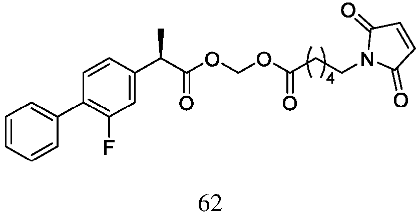

General Synthetic Method 6 was employed for preparation to yield a colorless liquid with a yield of 51.38%.

¹H NMR (400 MHz, Chloroform-d) δ 7.58 - 7.49 (m, 2H), 7.45 (dt, *J =* 6.9, 1.3 Hz, 1H), 7.44 - 7.32 (m, 3H), 7.18 - 7.07 (m, 2H), 6.67 (s, 2H), 5.75 (q, *J =* 5.6 Hz, 2H), 3.80 (q, *J* = 7.2 Hz, 1H), 3.48 (t, *J =* 7.2 Hz, 2H), 2.30 (t, *J =* 7.5 Hz, 2H), 1.67 - 1.59 (m, 3H), 1.59 - 1.52 (m, 5H), 1.34 - 1.22 (m, 3H).

¹H NMR (400 MHz, Chloroform-d) δ 7.58 - 7.49 (m, 2H), 7.45 (dt, *J =* 6.9, 1.3 Hz, 1H), 7.44 - 7.32 (m, 3H), 7.18 - 7.07 (m, 2H), 6.67 (s, 2H), 5.75 (q, *J =* 5.6 Hz, 2H), 3.80 (q, *J* = 7.2 Hz, 1H), 3.48 (t, *J =* 7.2 Hz, 2H), 2.30 (t, *J =* 7.5 Hz, 2H), 1.67 - 1.59 (m, 2H), 1.59 - 1.52 (m, 5H), 1.34 - 1.22 (m, 2H). (R.20.6, 21.11.2023)

### Example 61: Preparation of Compound 63

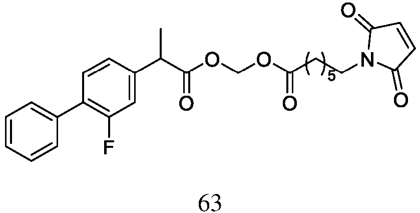

General Synthetic Method 6 was employed for preparation to yield a colorless liquid with a yield of 55.51%.

¹H NMR (400 MHz, Chloroform-d) δ 7.57 - 7.49 (m, 2H), 7.49 - 7.32 (m, 4H), 7.18 - 7.07 (m, 2H), 6.67 (s, 2H), 5.80 - 5.73 (m, 2H), 3.79 (q, J = 7.1 Hz, 1H), 3.52 - 3.43 (m, 2H), 2.36 - 2.25 (m, 2H), 1.63 - 1.51 (m, 8H), 1.35 - 1.19 (m, 3H).

### Example 62: Preparation of Compound 64

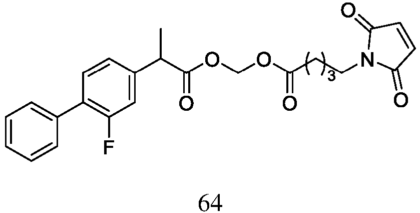

General Synthetic Method 6 was employed for preparation to yield a colorless liquid with a yield of 56.93%.

¹H NMR (400 MHz, Chloroform-*d*) δ 7.57 - 7.49 (m, 2H), 7.48 - 7.33 (m, 4H), 7.14 (dd, J = 7.9, 1.8 Hz, 1H), 7.11 (dd, J = 11.3, 1.8 Hz, 1H), 6.67 (s, 2H), 5.80 - 5.71 (m, 2H), 3.80 (q, J = 7.1 Hz, 1H), 3.53 - 3.46 (m, 2H), 2.39 - 2.31 (m, 2H), 1.59 (s, 3H), 1.55 (d, J = 7.0 Hz, 4H).

### Example 63: Preparation of Compound 65

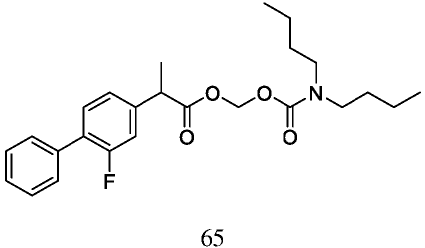

General Synthetic Method 2 was employed for preparation to yield a colorless oily product with a yield of 30.98%.

¹H NMR (600 MHz, Chloroform-*d*) δ 7.52 (dd, J = 7.1, 1.5 Hz, 2H), 7.43 (t, J = 7.6 Hz, 2H), 7.41 - 7.35 (m, 2H), 7.17 - 7.10 (m, 2H), 5.79 (s, 2H), 3.79 (q, J = 7.1 Hz, 1H), 3.28 - 3.08 (m, 4H), 1.55 (d, J = 7.1 Hz, 3H), 1.49 (p, J = 7.6 Hz, 2H), 1.39 (p, J = 7.4 Hz, 2H), 1.29 (p, J = 7.4 Hz, 2H), 1.25 - 1.18 (m, 2H), 0.91 (m, 6H).

### Example 64: Preparation of Compound 66

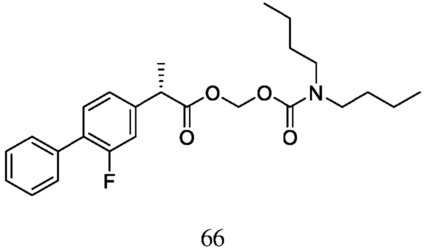

General Synthetic Method 2 was employed for preparation to yield a colorless oily product with a yield of 29.75%.

¹H NMR (600 MHz, Chloroform-*d*) δ 7.52 (dt, J = 8.1, 1.4 Hz, 2H), 7.42 (dd, J = 8.5, 6.9 Hz, 2H), 7.39 - 7.33 (m, 2H), 7.16 - 7.10 (m, 2H), 5.78 (s, 2H), 3.79 (q, J = 7.1 Hz, 1H), 3.27 - 3.16 (m, 2H), 3.11 (td, J = 7.3, 4.6 Hz, 2H), 1.54 (d, J = 7.2 Hz, 3H), 1.49 (p, J = 7.5 Hz, 2H), 1.39 (p, J = 7.5 Hz, 2H), 1.28 (q, J = 7.5 Hz, 2H), 1.21 (h, J = 7.5 Hz, 2H), 0.88 (dt, J = 22.8, 7.4 Hz, 6H).

### Example 65: Preparation of Compound 67

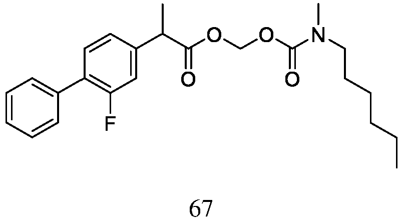

General Synthetic Method 2 was employed for preparation to yield a colorless oily product with a yield of 77.35%.

¹H NMR (600 MHz, Chloroform-d) δ 7.52 (dt, J = 8.0, 1.5 Hz, 2H), 7.43 (dd, J = 8.5, 6.9 Hz, 2H), 7.41 - 7.33 (m, 2H), 7.15 (dd, J = 7.9, 1.9 Hz, 1H), 7.11 (dd, J = 11.5, 1.8 Hz, 1H), 5.81 - 5.75 (m, 2H), 3.82 - 3.77 (m, 1H), 3.26 - 3.20 (m, 1H), 3.15 (h, J = 6.9 Hz, 1H), 2.85 (d, J = 45.8 Hz, 3H), 1.54 (d, J = 7.0 Hz, 4H), 1.49 (t, J = 7.2 Hz, 1H), 1.42 (t, J = 7.2 Hz, 1H), 1.26 (s, 4H), 1.21 (s, 1H), 0.86 (t, J = 6.8 Hz, 3H).

### Example 66: Preparation of Compound 68

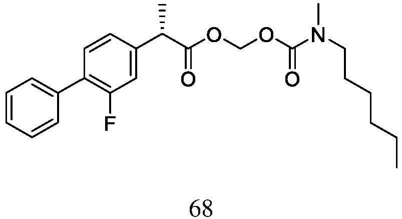

General Synthetic Method 2 was employed for preparation to yield a colorless oily product with a yield of 76.59%.

¹H NMR (600 MHz, Chloroform-*d*) δ 7.52 (dt, J = 8.1, 1.4 Hz, 2H), 7.46 - 7.33 (m, 4H), 7.18 - 7.09 (m, 2H), 5.82 - 5.73 (m, 2H), 3.78-3.80 (m, 1H), 3.25 - 3.20 (m, 1H), 3.15 (d, J = 8.0 Hz, 1H), 2.85 (d, J = 45.8 Hz, 3H), 1.42 (t, J = 7.2 Hz, 1H), 1.26 (s, 7H), 0.87 (t, J = 6.5 Hz, 3H).

¹H NMR (600 MHz, Chloroform-d) δ 7.52 (dt, *J* = 8.1, 1.4 Hz, 2H), 7.46 - 7.33 (m, 4H), 7.18 - 7.08 (m, 2H), 5.84 - 5.72 (m, 2H), 3.79 (dd, *J* = 7.4, 3.2 Hz, 1H), 3.25 - 3.08 (m, 2H), 2.85 (d, *J* = 45.8 Hz, 3H), 1.55 (d, *J* = 7.1 Hz, 3H), 1.51 - 1.39 (m, 2H), 1.24 (d, *J* = 23.8 Hz, 6H), 0.86 (t, *J* = 6.5 Hz, 3H). (R.20.6, 21.11.2023)

### Example 67: Preparation of Compound 69

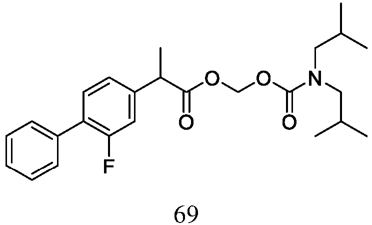

General Synthetic Method 2 was employed for preparation to yield a colorless oily product with a yield of 81.27%.

¹H NMR (600 MHz, DMSO-*d*₆) δ 7.52 (dt, J = 8.2, 1.5 Hz, 2H), 7.50 - 7.45 (m, 3H), 7.44 - 7.39 (m, 1H), 7.23 - 7.19 (m, 2H), 5.75 (d, J = 5.7 Hz, 1H), 5.67 (d, J = 5.9 Hz, 1H), 3.93 (q, J = 7.1 Hz, 1H), 3.02 - 2.90 (m, 4H), 1.86 (dt, J = 13.8, 7.0 Hz, 1H), 1.76 (dt, J = 13.8, 6.9 Hz, 1H), 1.43 (d, J = 7.1 Hz, 3H), 0.74 (dt, J = 33.3, 6.4 Hz, 12H).

### Example 68: Preparation of Compound 70

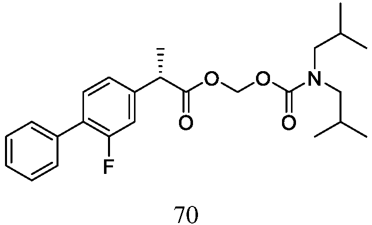

General Synthetic Method 2 was employed for preparation to yield a colorless oily product with a yield of 80.52%.

¹H NMR (600 MHz, DMSO-*d*₆) δ 7.54 - 7.46 (m, 5H), 7.42 - 7.38 (m, 1H), 7.23 - 7.19 (m, 2H), 5.75 (d, J = 5.7 Hz, 1H), 5.67 (d, J = 5.9 Hz, 1H), 3.93 (q, J = 7.1 Hz, 1H), 3.03 - 2.90 (m, 4H), 1.86 (dt, J = 13.6, 6.9 Hz, 1H), 1.75 (dq, J = 13.7, 6.9 Hz, 1H), 1.43 (d, J = 7.1 Hz, 3H), 0.74 (dt, J = 33.3, 6.5 Hz, 12H).

### Test Examples

### Test Example 1: In Vivo Pharmacokinetic Assay in Rats

Experimental method: Adult female Sprague Dawley rats, aged 8-10 weeks and weighing 200 ± 20 g, were randomly grouped, with 3 rats in each group. Rats in each of the groups were administered by gavage at a dose of 50 mg/kg of flurbiprofen and an equimolar dose of other example compounds to 50 mg/kg of flurbiprofen, respectively. After gavage administration, blood was collected at 0.0833 h, 0.25 h, 0.5 h, 1 h, 2 h, 3 h, 4 h, 6 h, 9 h, 12 h, and 24 h into sodium heparin anticoagulant tubes, respectively. The blood was collected by the following method: 250 µL of blood were collected from the orbital venous plexuses of the rats. After blood collection, the blood samples were centrifuged at 12000 rpm in a centrifuge for 5 min. All plasma was aspirated into a 1.5-mL centrifuge tube, and the plasma was stored in a refrigerator at -80°C.

The pharmacokinetic parameters were determined by the external standard curve method. Sample processing: after the cryopreserved samples were removed and restored to room temperature, the samples were vortexed evenly and then diluted two folds with methanol; the sample solution was diluted 5 folds by adding a mixed extractant of 0.5% formic acid-containing acetonitrile: methanol = 7:3, and subjected to vortex oscillation for 1 min. Subsequently, the resultant was centrifuged at 4°C and 12000 rpm for 10 min, and then 5 µL of the supernatant was measured for assay. The pharmacokinetic parameters in the Examples were calculated using the Winnonlin software. The results were as shown in Table 1.

The results showed that the exposure or half-life of the metabolites of the above compounds of the present disclosure in the rat plasma was significantly increased as compared to flurbiprofen.

**Table 1. Pharmacokinetic Parameters for Test Drugs**

| **Compounds** | **Prototype Drug** | | | **Flurbiprofen** | | |
|---|---|---|---|---|---|---|
| | AUC | Cₘₐₓ | t_{1/2} | AUC | Cₘₐₓ | t_{1/2} |
| Flurbiprofen | -- | -- | -- | 752644±83652 | 110628±9471 | 3.74±1.00 |
| Compound 33 | 8.75±1.53 | 1.56+0.509 | 9.08±1.44 | 858774±351764 | 73992±30524 | 8.50+3.78 |
| Compound 1 | - | - | - | 758774±351764 | 63992±30524 | 8.50+3.78 |
| Compound 2 | 183±85.9 | 27.1±7.20 | 14.0±9.88 | 1644486±155721 | 402771±35343 | 3.80±0.753 |
| Compound 9 | | | | 914578±259601 | 88698±6884 | 6.58±1.46 |
| Compound 16 | - | - | - | 973908±93514 | 89096±8691 | 5.28±0.553 |
| Compound 18 | - | - | - | 990213486222 | 86636±9073 | 7.61±1.95 |
| Compound 34 | - | - | - | 858829±158715 | 72804±20243 | 8.45±4.54 |
| Compound 35 | - | - | - | 796631±71412 | 62248±1729 | 7.34±3.15 |
| Compound 25 | 63012±11787 | 3865±178 | 8.10±4.42 | 7901097±2231756 | 593596±134139 | 5.14±0.530 |
| Compound 36 | - | - | - | 844289±132155 | 83071±5316 | 6.54±0.693 |
| Compound 27 | | | | 896631±71412 | 72248±1729 | 7.34±3.15 |
| Compound 46 | - | - | - | 752414±86592 | 68454±11858 | 4.89±0.02 |
| Compound 47 | - | - | - | 884288±49939 | 77206±11698 | 5.44±0.10 |

### Test Example 2: Anti-Inflammatory Effect on Toe Swelling in Experimental Rats

Experimental method: Male SD rats weighing 150-160 g were randomly grouped, with 10 rats in each group. The rats were intraperitoneally injected with saline, the compounds of the present disclosure, and the control drug flurbiprofen or flurbiprofen axetil respectively, all of which were administered at a dose of 10 mg/kg and a dosing volume of 10 µL/g. At 1 h after administration, 100 µL of 1% λ-carrageenan was injected into the right hind toe of each rat to induce inflammation. Afterwards, the paw volume was measured every 1 h using a paw volume meter to calculate the swelling inhibition rate.

The results showed that all the compounds of the present disclosure in Table 2 exhibited a better anti-inflammatory effect as compared to flurbiprofen.

**Table 2. Anti-Inflammatory Effect on λ-Carrageenan-Induced Rat Toe Swelling**

| Groups | Dose (mg/kg) | Toe Swelling Inhibition Rate at 6 h | Toe Swelling Inhibition Rate at 24 h |
|---|---|---|---|
| Model control group | Equal volume of saline | -- | -- |
| Flurbiprofen | 10.0 | 35.50%** | 29.95%** |
| Flurbiprofen axetil | 10.0 | 35.53%** | 22.85%** |
| Compound 33 | 10.0 | 55.52%*** | 35.83%*** |
| Compound 2 | 10.0 | 42.94%** | 34.09%** |
| Compound 3 | 10.0 | 45.29%* | 28.10%* |
| Compound 7 | 10.0 | 47.88%*** | 21.48%* |
| Compound 13 | 10.0 | 46.03%** | 37.01%** |
| Compound 20 | 10.0 | 52.72%*** | 45.00%*** |
| Compound 35 | 10.0 | 54.63% | 49.84% |
| Compound 36 | 10.0 | 45.29%* | 28.10% |
| Compound 41 | 10.0 | 43.04%** | 32.23%** |
| Compound 29 | 10.0 | 45.82%*** | 29.56%** |
| Compound 31 | 10.0 | 45.35%** | 31.95%** |
| Compound 47 | 10.0 | 49.26%*** | 33.85%** |
| Compound 48 | 10.0 | 44.10%** | 33.85%** |
| Compound 53 | 10.0 | 69.04%*** | 75.24%*** |
| Compound 55 | 10.0 | 36.66%* | 41.39%** |
| Compound 57 | 10.0 | 43.84%** | 43.42%** |
| Compound 58 | 10.0 | 43.64%** | 47.65%*** |
| Compound 61 | 10.0 | 61.72%*** | 52.93%*** |
| Compound 63 | 10.0 | 47.92%*** | 32.31%** |
| Compound 64 | 10.0 | 54.28%*** | 40.36%*** |
| Compound 65 | 10.0 | 43.08%*** | 43.84%*** |
| Compound 67 | 10.0 | 48.62%*** | 57.31%*** |
| Compound 69 | 10.0 | 65.91%*** | 70.62%*** |

| | | | |
|---|---|---|---|
| * denoted that the P value was less than 0.05, ** denoted that the P value was less than 0.01, *** denoted that the P value was less than 0.001. | | | |

### Test Example 3: Effects on Dry Yeast-Induced Fever in Rats

Experimental method: Male SD rats, aged 4 weeks, were randomly grouped, with 10 rats in each group. Under the ambient temperature of 20°C to 21°C and the humidity of 40% to 70%, the body temperatures were screened and the average body temperature was at about 37.4°C, with the elimination rate of about 10%. The rats were intraperitoneally injected with saline, the compounds of the present disclosure or the control drug flurbiprofen respectively, all of which were administered at a dose of 10 mg/kg and a dosing volume of 10 µL/g. At 20 min after administration, 20% active dry yeast suspension was injected subcutaneously. After modeling, the rectal temperatures were measured at 4 h and 8 h.

The results showed that all the compounds of the present disclosure in Table 3 exhibited a better antipyretic effect as compared to flurbiprofen.

**Table 3. Antipyretic Effect on Dry Yeast-Induced Rat Fever Models**

| Groups | Dose (mg/kg) | Temp. at 4 h After Dosing (°C) | Temp. at 8 h After Dosing (°C) |
|---|---|---|---|
| Model control group | Equal volume of saline | 38.58 ± 0.42 | 39.52 ± 0.34 |
| Flurbiprofen | 10.0 | 37.49 ± 0.43 | 37.67 ± 0.39 |
| Compound 33 | 10.0 | 37.18 ± 0.18 | 37.27 ± 0.28 |
| Compound 2 | 10.0 | 37.10 ± 0.17 | 37.32 ± 0.27 |
| Compound 9 | 10.0 | 37.17 ± 0.23 | 37.10 ± 0.37 |
| Compound 13 | 10.0 | 37.10 ± 0.172 | 37.32 ± 0.27 |
| Compound 15 | 10.0 | 37.16 ± 0.16 | 37.27 ± 0.24 |
| Compound 20 | 10.0 | 37.09 ± 0.25 | 37.27 ± 0.26 |
| Compound 36 | 10.0 | 37.38 ± 0.31 | 37.24 ± 0.16 |
| Compound 41 | 10.0 | 37.19 ± 0.35 | 37.30 ± 0.31 |
| Compound 35 | 10.0 | 37.15 ± 0.21 | 37.29 ± 0.30 |
| Compound 29 | 10.0 | 37.12 ± 0.13 | 37.35 ± 0.23 |
| Compound 31 | 10.0 | 37.12 ± 0.30 | 37.22 ± 0.30 |
| Compound 47 | 10.0 | 37.17 ± 0.27 | 37.26 ± 0.22 |
| Compound 48 | 10.0 | 37.11 ± 0.23 | 37.21 ± 0.25 |
| Compound 53 | 10.0 | 37.04 ± 0.20 | 37.02 ± 0.30 |
| Compound 61 | 10.0 | 37.14 ± 0.29 | 37.06 ± 0.32 |
| Compound 63 | 10.0 | 37.05 ± 0.18 | 37.14 ± 0.25 |
| Compound 64 | 10.0 | 37.09 ± 0.16 | 37.18 ± 0.26 |
| Compound 65 | 10.0 | 37.10 ± 0.20 | 37.24 ± 0.23 |
| Compound 67 | 10.0 | 37.17 ± 0.19 | 37.16 ± 0.20 |
| Compound 69 | 10.0 | 37.09 ± 0.18 | 37.13 ± 0.27 |

### Test Example 4: Analgesic and Anti-Inflammatory Effects on Collagen-Induced Rheumatoid Arthritis (CIA)

Experimental method: Female Lewis rats, aged 6-8 weeks, were randomly grouped, with 8 rats in each group. Under the ambient temperature of 20.5°C to 24.5°C and the humidity of 40% to 70%, the light period was 12-h bright and 12-h dark. Type II collagen was mixed with Freund's incomplete adjuvant (IFA) at a ratio of 1:1 and fully emulsified on ice before use. 100 µL of the mixed solution was injected intradermally at the part 2 to 3 cm from the base of the rat tail, and 50 µL of the mixed solution was injected into the bases of both hind limbs separately. The rats in the blank control group were injected with an equal volume of saline. IFA was used for re-immunization 7 days later. The rats were observed for the incidence of disease every day after the second immunization. Rats with a total score of more than 2 on both hind limbs were selected and randomly divided into a model group, a flurbiprofen group, a compound 33 group, a compound 2 group, a compound 13 group, a compound 20 group, and a compound 35 group according to the body weight, score and paw volume, and furthermore a blank control group was set up, with 8 rats in each group. The dosing volume was 10 mg/kg, and the dosing time was 14 days. A corresponding volume of solvent was administered in the model group and the blank control group.

Test indicators: body weight, arthritis score, hind limb paw volume, and mechanical foot threshold (MWT).

Standard for arthritis score: 0 = normal; 1 = erythema and slight swelling observed at the ankle joint; 2 = erythema and slight swelling observed from the ankle joint to the metatarsal joint or the metacarpal joint; 3 = erythema and moderate swelling observed from the ankle joint to the metatarsophalangeal joint or the metacarpal joint; 4 = erythema and severe swelling observed from the ankle joint to the metatarsal joint; the total score of all (four) paws was calculated, and the sum of the arthritis indices was up to 16 per rat.

### Test results:

### 1. Effects of test drugs on body weight of rats with CIA:

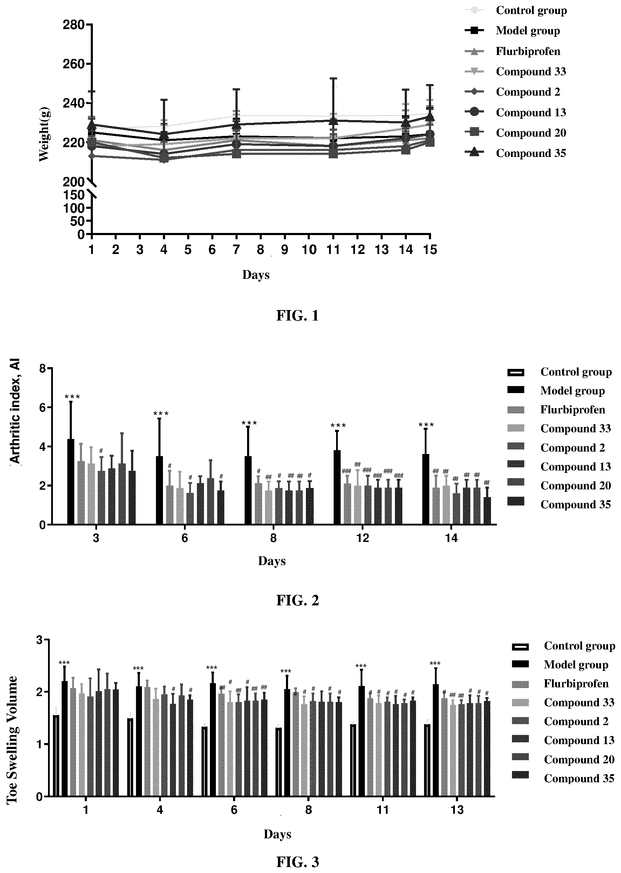

The results showed that there were no significant changes in the body weight of rats in each of groups during test, and the test drugs had no significant effect on the body weight of rats (Mean ± SD). The results were shown in FIG. 1.

### 2. Effects of test drugs on arthritis scores of rats with CIA:

The results showed that the scores of rats in the model group were significantly higher than those of the rats in the blank control group during administration, and the arthritis scores of rats with CIA could be significantly reduced after 14-day intervention with each of test drugs (FIG. 2, *P* < 0.01), indicating that the test drugs could significantly reduce the arthritis scores of rats with CIA.

### 3. Effects of test drugs on hind limb paw volume of rats with CIA:

After 14-day intervention with each of the test drugs, all of the test drugs showed a significant effect of reducing the paw volume of the left hind limbs of rats with CIA (*P* < 0.05 or 0.01). Compared with the model group, the paw volumes of the left lower limbs of rats with CIA were significantly reduced during the treatment with test drugs (FIG. 3). The groups of compound 33, compound 2, compound 13, compound 20, and compound 35 showed better effects as compared to the flurbiprofen group.

### 4. Effect of test drugs on mechanical withdrawal threshold (MWT) of the hind limbs of rats with CIA:

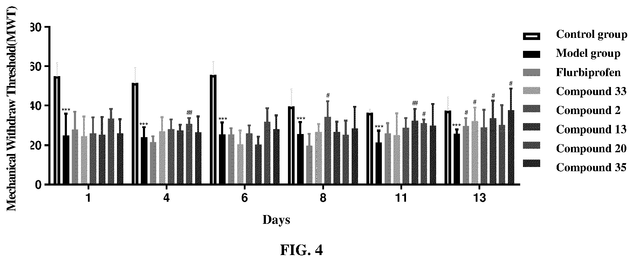

After successful modeling, the mechanical withdrawal threshold (MWT) of the left lower limbs of rats with CIA was significantly reduced as compared to the blank control group. After 13-day intervention and treatment with the test drugs, all the test drugs showed a significant effect of increasing MWT (FIG. 4, *P* < 0.05), among which the groups of compound 33, compound 2, compound 13, compound 20, and compound 35 showed slightly better or better effects as compared to the flurbiprofen group.

### Test Example 5: Antitumor Potency

Experimental method: The antitumor potency was tested by the MTT method. Tumor cells at the logarithmic phase were collected. The concentration of the cell suspension was adjusted. 100 µL of the cell suspension was added to each well. Cell plating was carried out and the cell density was adjusted to 1000 to 10000/well (the marginal wells were filled with sterile PBS). The cells were incubated at 5% CO₂ and 37°C until the cells adhered to the wall (96-well flat-bottomed plate). Drugs of different concentration gradients were added, 100 µL per well. Four replicates were set up. Under the conditions of 5% CO₂ and 37°C, the cells were incubated for 72 h and observed under an inverted microscope. The prepared MTT solution (5 mg/mL) was added to the 96-well plate, 20 µL per well, mixed well, and incubated for 4 h under the conditions of 37°C and 5% CO₂. Afterwards, the liquid in the plate was discarded. 150 µL of DMSO was added to each well and oscillated for 3 min on a microplate reader. The OD value (optical density) was detected at 490 nm.

The inhibition rate of the drug against the tumor cell growth was calculated according to the following formula: cell viability (%) = (OD value in the treatment group/OD value in the control group) × 100%

### Experimental results:

**Table 4. Antitumor Test Results (IC₅₀ value, unit: µm)**

| Tumor Cells | Eca-109 | MM.1S | NUGC-4 | U87 | U251 | H460 |
|---|---|---|---|---|---|---|
| Flurbiprofen | 85.54 | >100 | >100 | >100 | 34.12 | >100 |
| Compound 33 | >100 | >100 | >100 | >100 | >100 | >100 |
| Compound 2 | 55.73 | 41.70 | 59.96 | >100 | >100 | 90.03 |
| Compound 13 | 30.69 | 5.39 | 40.10 | 80.46 | 73.86 | 47.94 |
| Compound 20 | 33.18 | 42.75 | 50.43 | 54.63 | 5.84 | 35.89 |
| Compound 35 | 29.62 | 45.73 | 41.78 | 60.31 | 7.33 | 32.58 |

## Claims

**1.** A compound represented by formula (I), or an optical isomer thereof, or a pharmaceutically acceptable salt thereof, or a solvate thereof (such as a hydrate), or an inclusion compound thereof, or a racemate thereof, or a co-crystal thereof, or an isotopically labelled compound thereof, or a nitrogen oxide thereof, 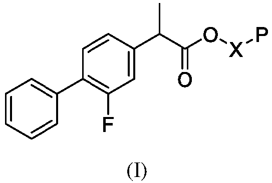 wherein
X is alkylene;
P is 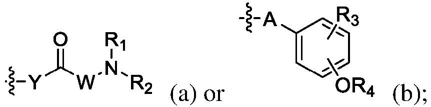 in formula (a),
Y is a single bond, -O-, -S-, or -NH-;
W is a single bond or alkylene;
R₁ and R₂ are the same or different and are each independently selected from -H, optionally substituted alkyl, and optionally substituted aryl, or R₁ and R₂ are linked to each other and jointly form, together with the nitrogen atoms to which they are linked, optionally substituted aliphatic heterocyclyl or 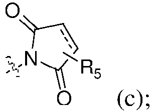
there are one or two R₅, each independently selected from -H and alkyl;
in formula (c), a dashed line indicates a single bond or null;
in formula (b),
A is a single bond, -O-(C=O)- or -O-(C=O)-alkylene;
R₃ is an optional group;
there is one or more R₃, if present, and each independently alkyl, hydroxy, carboxy, hydroxy-substituted alkyl, alkoxy, or -N(R₅)(R₆), and R₅ and R₆ are the same or different and are each independently alkyl;
R₄ is selected from -H, alkyl, and alkylacyl; or
R₃ and -OR₄ are linked to each other to form an aliphatic heterocyclic ring;
the "optionally substituted" refers to being unsubstituted or substituted with one or more substituents, wherein the substituents in the "optionally substituted alkyl", "optionally substituted aryl", and "optionally substituted aliphatic heterocyclyl" are each independently selected from hydroxy, amino, carboxy, halogen, nitro, cyano, alkyl, alkylthio, alkylacyl, and hydroxy-substituted aryl.

**2.** The compound represented by formula (I), or the optical isomer thereof, or the pharmaceutically acceptable salt thereof, or the solvate thereof (such as a hydrate), or the inclusion compound thereof, or the racemate thereof, or the co-crystal thereof, or the isotopically labelled compound thereof, or the nitrogen oxide thereof according to claim 1, wherein
the alkyl moieties in the "alkyl", "alkylacyl", "hydroxy-substituted alkyl", "alkoxy", and "alkylthio" are each independently C₁₋₂₀ linear or branched alkyl, optionally C₁₋₁₇ linear or branched alkyl, optionally C₁₋₁₀ linear or branched alkyl, optionally C₁₋₇ linear or branched alkyl, optionally methyl, ethyl, n-propyl, isopropyl, n-butyl, isobutyl, tert-butyl, sec-butyl, n-pentyl, isopentyl, neopentyl, tert-pentyl, n-hexyl, isohexyl, heptyl, n-octyl, n-nonyl, n-decyl, undecyl, dodecyl, tridecyl, tetradecyl, pentadecyl, or hexadecyl;
optionally, the "alkylene" is C₁₋₂₀ linear or branched alkylene, optionally C₁₋₁₇ linear or branched alkylene, optionally C₁₋₁₀ linear or branched alkylene, optionally C₁₋₈ linear or branched alkylene, optionally C₁₋₆ linear or branched alkylene, optionally C₁₋₃ linear or branched alkylene, optionally methylene, ethylene, isoethylidene, n-propylene, isopropylene, n-butylidene, isobutylidene, tert-butylidene, sec-butylidene, n-pentylidene, isopentylidene, neopentylidene, tert-pentylidene, n-hexylidene, isohexylidene, heptylidene, n-octylidene, n-nonylidene, or n-decylidene;
optionally, the aliphatic heterocyclic ring in the "aliphatic heterocyclic ring" or "aliphatic heterocyclyl" is a C₃₋₈ (preferably C₄₋₆) aliphatic heterocyclic ring containing 1 to 3 heteroatoms selected from O, N, and S on the ring, optionally dioxolane, aziridinyl, azetidinyl, tetrahydropyrrolyl, morpholinyl, piperidinyl, or piperazinyl;
optionally, the "aryl" or "hydroxy-substituted aryl" is a 6- to 10-membered monocyclic or bicyclic fused aromatic ring group; optionally phenyl or naphthyl;
optionally, the present disclosure relates to the compounds as described above and any appended definitions, wherein a 3-fluorobenzenepropanoate moiety is in an S configuration or an R configuration.

**3.** The compound represented by formula (I), or the optical isomer thereof, or the pharmaceutically acceptable salt thereof, or the solvate thereof (such as a hydrate), or the inclusion compound thereof, or the racemate thereof, or the co-crystal thereof, or the isotopically labelled compound thereof, or the nitrogen oxide thereof according to claim 1 or 2, wherein R₁ and R₂ are the same or different and are each independently selected from -H, n-propyl, isopropyl, 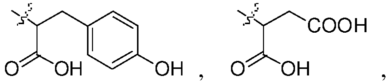 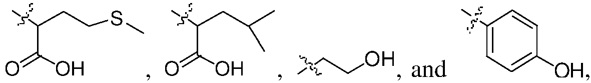 or R₁ and R₂ are linked to each other and jointly form, together with the nitrogen atoms to which they are linked, the following group: heterocyclopropan-1-yl, azetidin-1-yl, tetrahydropyrrol-1-yl, morpholin-1-yl, piperidin-1-yl, or piperazin-1-yl, 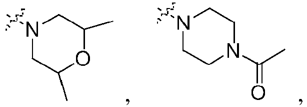 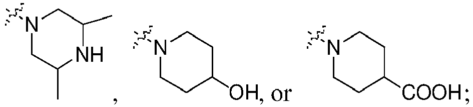 optionally, when R₃ and -OR₄ are linked to each other to form an aliphatic heterocyclic ring, the aliphatic heterocyclic ring is dioxolane.

**4.** The compound represented by formula (I), or the optical isomer thereof, or the pharmaceutically acceptable salt thereof, or the solvate thereof (such as a hydrate), or the inclusion compound thereof, or the racemate thereof, or the co-crystal thereof, or the isotopically labelled compound thereof, or the nitrogen oxide thereof according to any one of claims 1 to 3, wherein
the pharmaceutically acceptable salt is an inorganic acid salt or an organic acid salt, preferably selected from hydrochloric acid, sulfuric acid, phosphoric acid, nitric acid, methanesulfonic acid, tartaric acid, formic acid, acetic acid, salicylic acid, citric acid, succinic acid, fumaric acid, or benzoic acid.

**5.** The compound represented by formula (I), or the optical isomer thereof, or the pharmaceutically acceptable salt thereof, or the solvate thereof (such as a hydrate), or the inclusion compound thereof, or the racemate thereof, or the co-crystal thereof, or the isotopically labelled compound thereof, or the nitrogen oxide thereof according to any one of claims 1 to 4, which is selected from the following compounds, or optical isomers thereof, or solvates thereof, or inclusion compounds thereof, or racemates thereof, or co-crystals thereof, or isotopically labelled compounds thereof, or nitrogen oxides thereof:

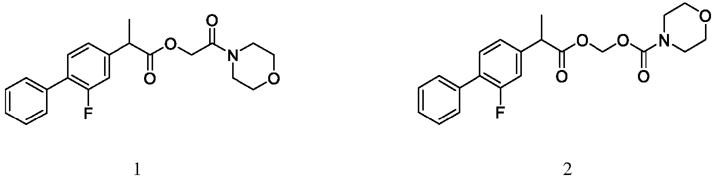

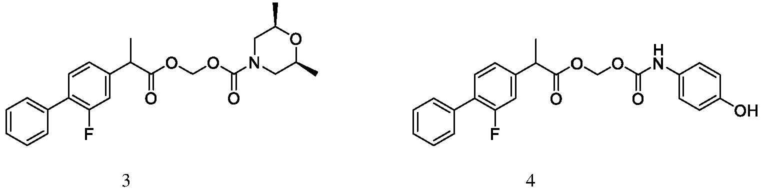

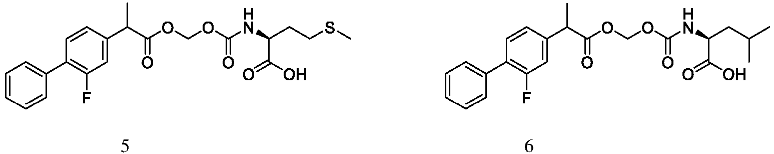

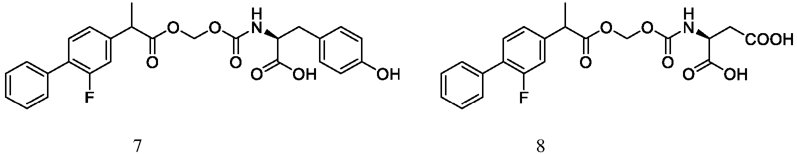

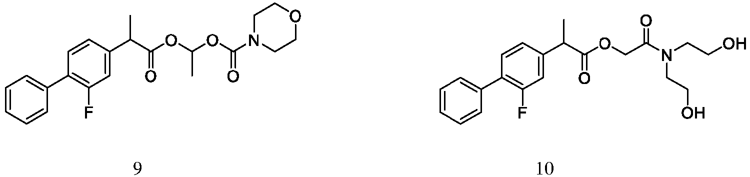

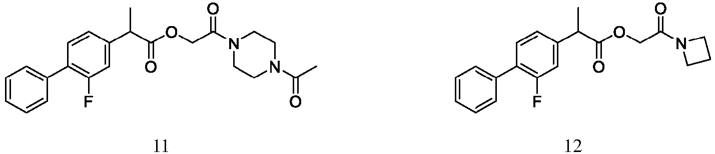

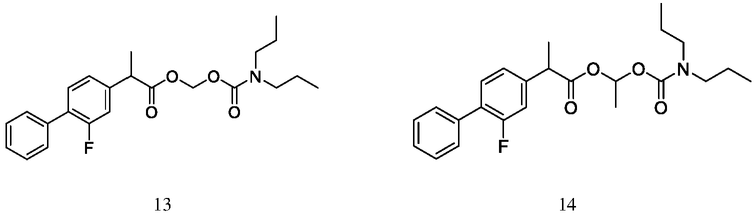

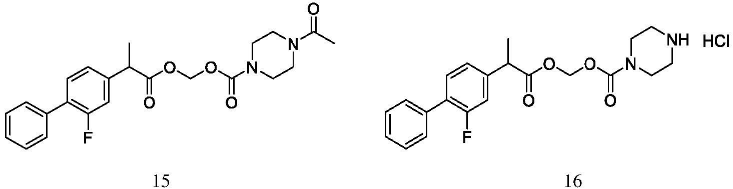

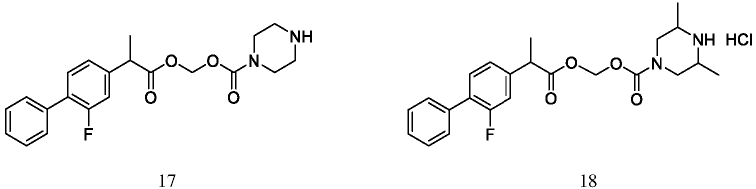

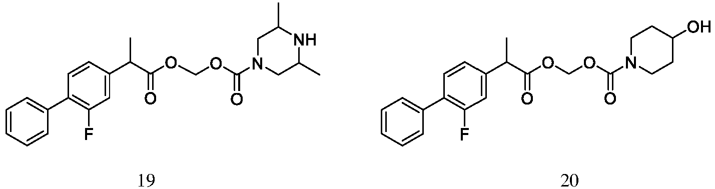

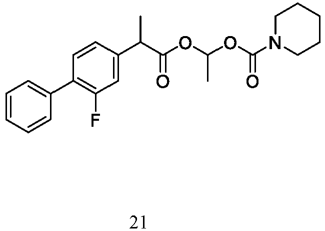

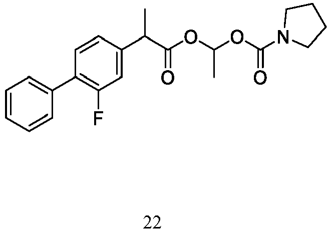

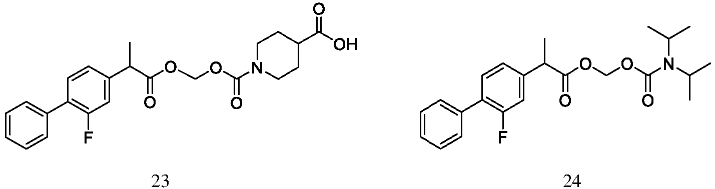

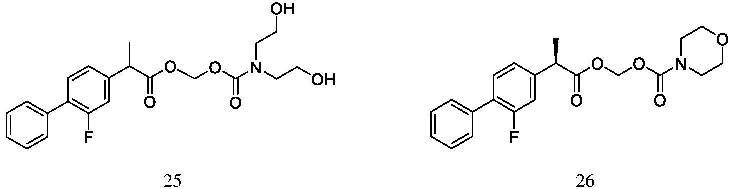

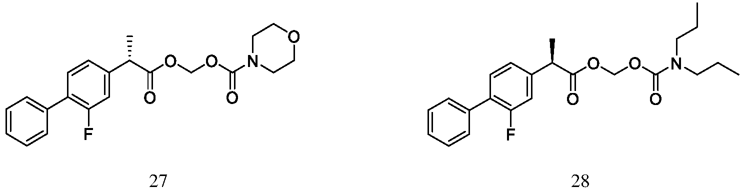

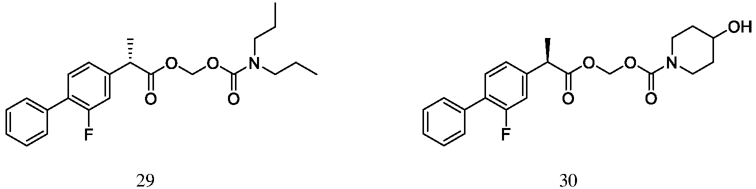

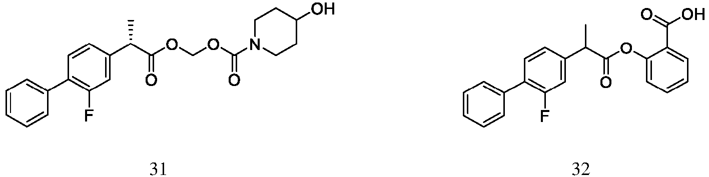

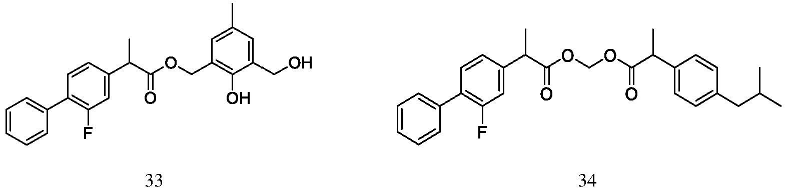

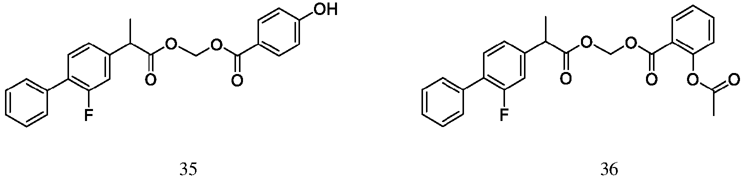

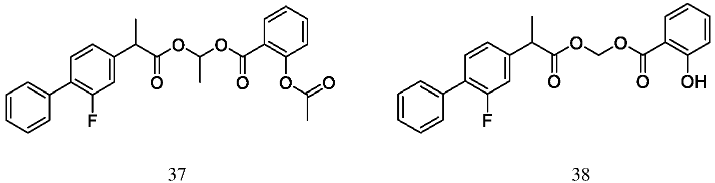

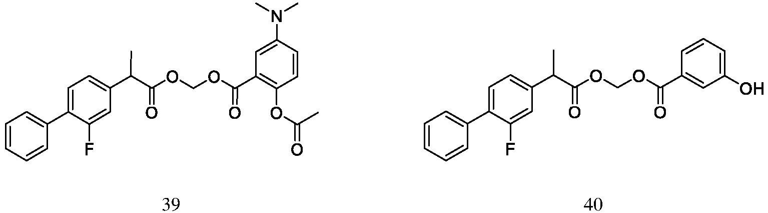

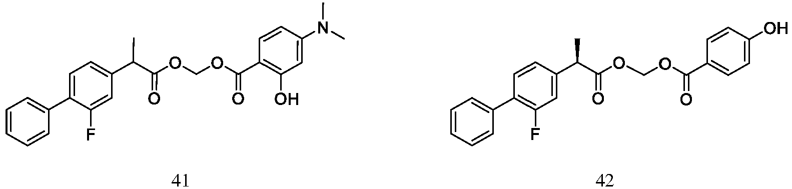

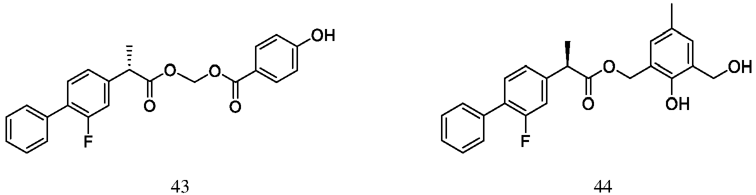

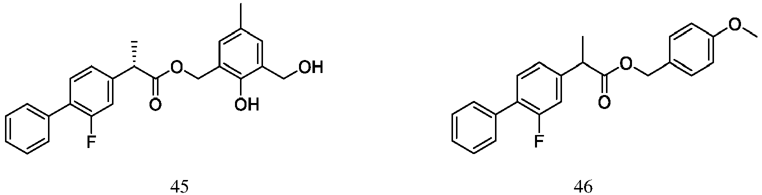

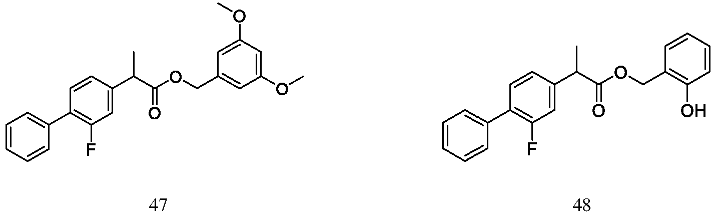

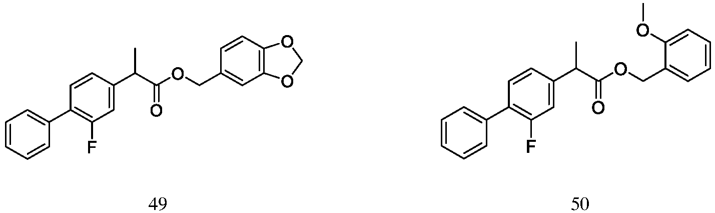

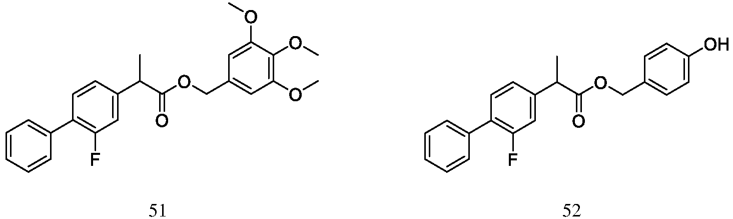

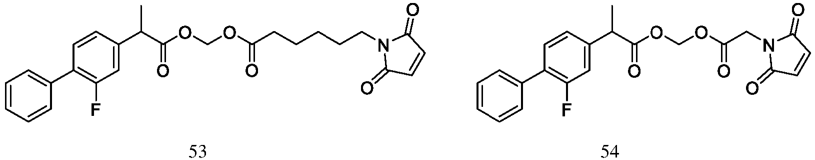

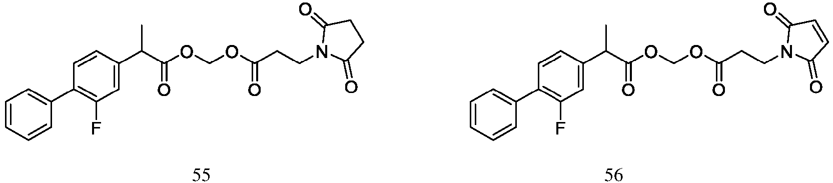

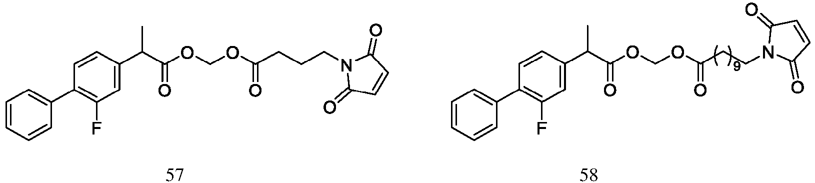

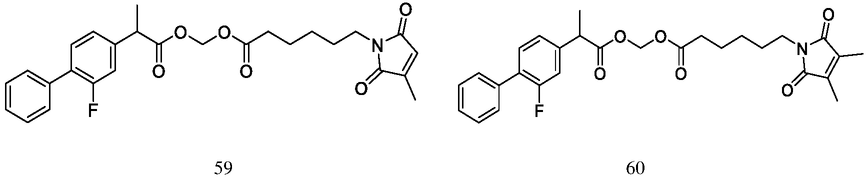

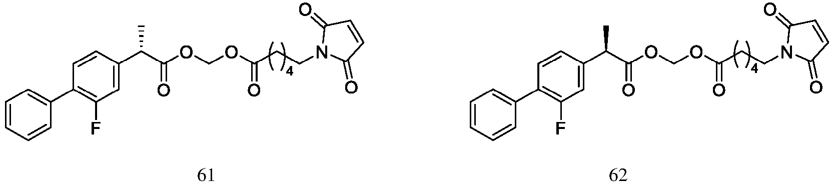

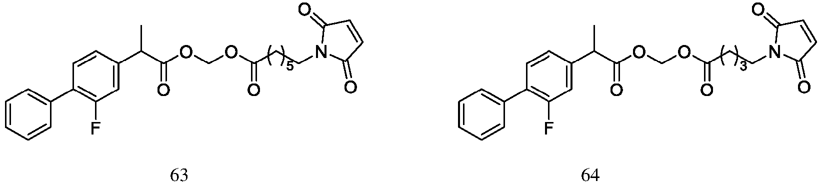

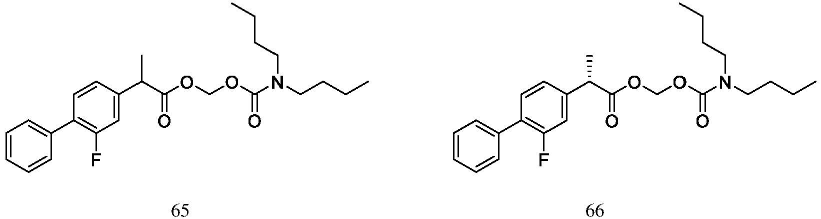

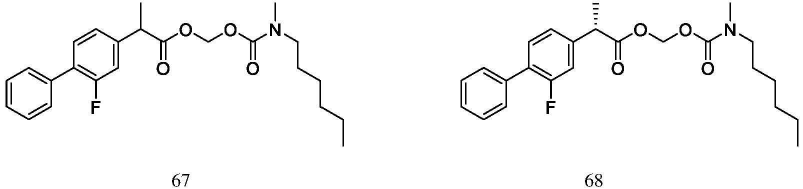

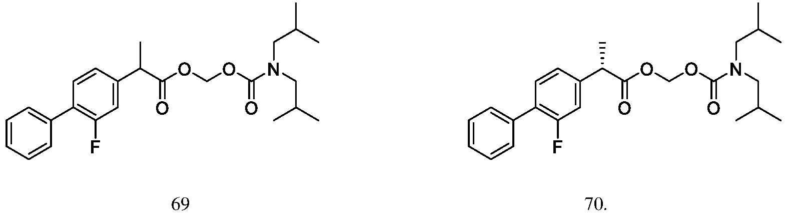

**5.** A preparation method for the compound represented by formula (I), or the optical isomer thereof, or the pharmaceutically acceptable salt thereof, or the solvate thereof (such as a hydrate), or the inclusion compound thereof, or the racemate thereof, or the co-crystal thereof, or the isotopically labelled compound thereof, or the nitrogen oxide thereof according to any one of claims 1 to 4, wherein
in formula (I), when P is 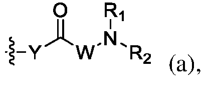 the preparation method comprises a step of preparing formula (II) from a compound of formula (A) and a compound of formula (B) by method A:
method A: preparing formula (II) by subjecting formula (A) and formula (B) to a substitution reaction; 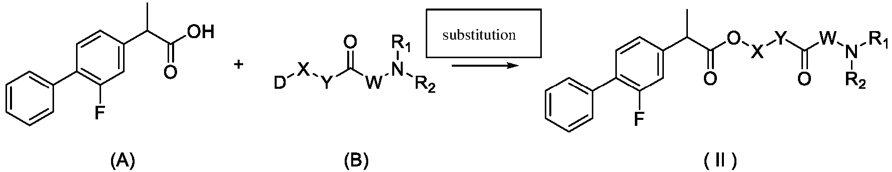 wherein
formula (A) is an R configuration, an S configuration or a racemate;
in formula (B) and formula (II), X, Y, W, R₁ and R₂ are as described in formula (I);
in formula (B), D is selected from Cl, Br, I, OTs, and OMs;
each Y is -O-, -S-, or -NH-, wherein R₁ and R₂ are as described in formula (I);
optionally, formula (II) is prepared by subjecting formula (A) and formula (B) to a substitution reaction in a suitable solvent at a suitable temperature under catalysis of a base;
optionally, in the method A, the base used for the substitution reaction is one or more selected from pyridine or triethylamine or N,N-diisopropylethylamine or 1,8-diazabicyclo[5.4.0]undec-7-ene or potassium carbonate or cesium carbonate or sodium hydride or lithium bis(trimethylsilyl)amide; optionally, the solvent for the substitution reaction is one or more selected from DMF or DMAC or dimethyl sulfoxide or NMP or DCM or tetrahydrofuran or acetonitrile or ethyl acetate or isopropyl acetate or dioxane or acetone; optionally, the temperature for the substitution reaction is 0°C to 100°C;
optionally, formula (B) can be prepared by, but not limited to, one of the following steps 1 to 3:
step 1: 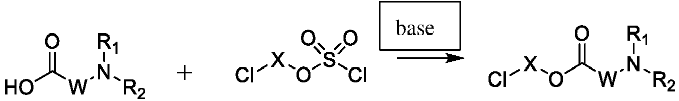 at room temperature, a saturated sodium bicarbonate solution and tetrabutylammonium hydrogen sulfate at a catalytic amount are added to a solution of an acid in dichloromethane that is being vigorously stirred, and then chloromethyl chlorosulfonate is added; after the reaction is completed, the reaction solution is layered, and the organic phases are collected, dried over anhydrous sodium sulfate, evaporated to dryness via rotary evaporation under reduced pressure, and purified by column chromatography to yield the compound of formula (B); and/or
step 2: 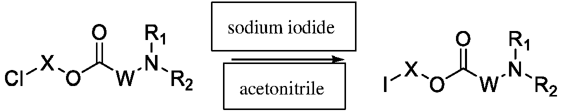 a chlorination reagent and sodium iodide (or potassium iodide) are refluxed in acetonitrile (or acetone); after the reaction is completed, the solvent is removed under reduced pressure, an organic solvent and water are added; the reaction solution is layered, and the organic phases are collected and dried over anhydrous sodium sulfate; and the solvent is removed under reduced pressure to yield a corresponding iodinated compound of formula (B);
optionally, when W is absent, formula (B) is synthesized by step 3;
or step 3: 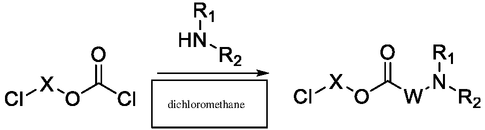
at 0°C, a corresponding secondary amine is added to a solution of chloromethyl chloroformate in dichloromethane; after the reaction is completed, the reaction solution is filtered, and the organic phases are washed with 1N hydrochloric acid and dried over anhydrous sodium sulfate; and the solvent is removed under reduced pressure to yield the compound of formula (B);
or when W is a single bond, the preparation method comprises preparing formula (II) from formula (A), formula (C) and formula (M) by method B;
method B: ① preparing formula (D) by subjecting formula (A) and formula (C) to a condensation reaction; ② preparing formula (E) by de-protecting formula (D); and ③ preparing formula (II) by condensing formula (E) with formula (M); 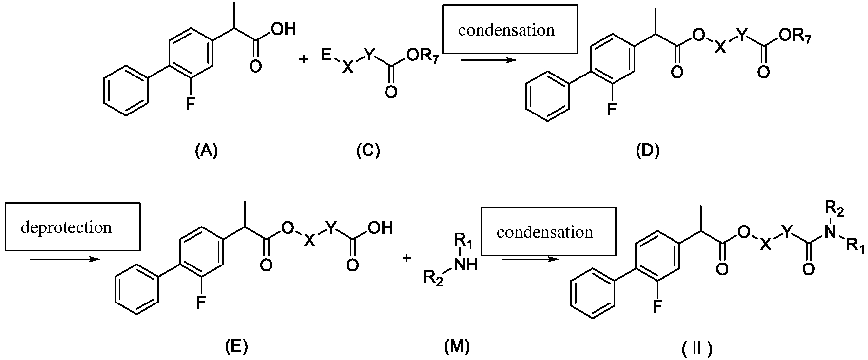 wherein
Y is a single bond, and X, R₁ and R₂ are as described in formula (I);
formula (A) is an R configuration, an S configuration or a racemate;
in formula (C), R₇ is methyl, ethyl, tert-butyl, benzyl, or p-methoxybenzyl; and E is -OH;
or when P is 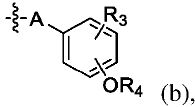
the preparation method comprises preparing formula (III) from a compound of formula (A) and a compound of formula (F) by method C;
method C: preparing formula (III) by subjecting formula (A) and formula (F) to a condensation reaction; 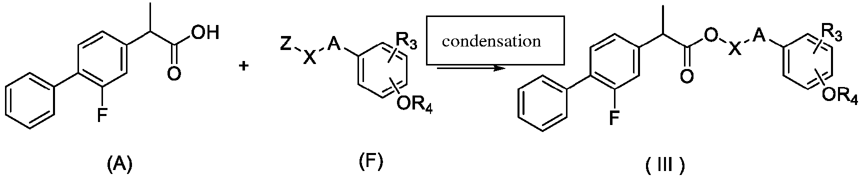 wherein
in formula (F), A is a single bond, and X, R₃ and R₄ are as described in formula (I);
in formula (F), Z is selected from -OH, -NH₂, -SH, and -NH-;
optionally, in the method B and method C, a condensing agent for the condensation reaction is one or more selected from DCC, DIC, EDCI, HATU, HBTU, CDI, HCTU, TBTU, TSTU, TNTU, HAPyU, HBPyU, BOP, PyBOP, PyAOP, DPPCl, DECP, DPPA, MPTA, or BOPCl, optionally a solvent for the condensation reaction is one or more selected from dichloromethane, dimethyl sulfoxide, N,N-dimethylformamide, hexamethylphosphoric triamide, or acetonitrile; optionally, the temperature for the condensation reaction is 0°C to 100°C;
or the preparation method comprises preparing a compound of formula (III) from formula (A) by method D:
method D: 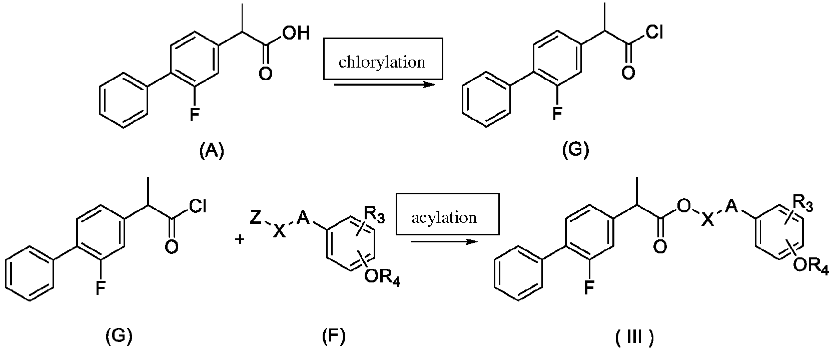 wherein
in formula (F), A is a single bond, and X, R₃, R₄ and Z are as described above;
or the preparation method comprises preparing a compound of formula (III) from formula (A) and formula (H) by method E:
method E: 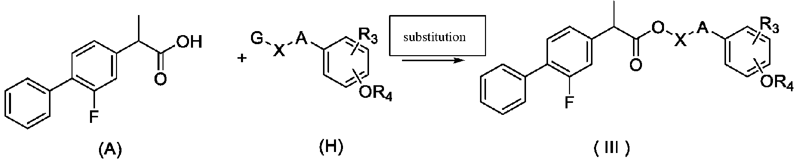 wherein
in formula (H), A is -O-(C=O)-; G is Cl, Br, I, OMs, or OTs; and X, R₃ and R₄ are as described in formula (I).

**7.** A pharmaceutical composition, comprising the compound represented by formula (I), or the optical isomer thereof, or the pharmaceutically acceptable salt thereof, or the solvate thereof (such as a hydrate), or the inclusion compound thereof, or the racemate thereof, or the co-crystal thereof, or the isotopically labelled compound thereof, or the nitrogen oxide thereof as described above, and a pharmaceutically acceptable excipient,
optionally, the pharmaceutically acceptable excipient is selected from fillers, disintegrants, lubricants, glidants, effervescent agents, flavoring agents, preservatives, solubilizers, cosolvents, antioxidants, anti-photolysis agents, pH regulators, emulsifiers, antibacterial preservatives, topical analgesics, complexing agents, non-aqueous solvents, coating materials or other vehicles;
optionally, as the pharmaceutically acceptable excipient, the fillers comprise a composition of one or more of lactose, mannitol, and calcium carbonate; the binders comprise a composition of one or more of sucrose, starch, povidone, and sodium carboxymethyl cellulose; the disintegrants comprises a composition of one or more of starch, cross-linked povidone, cross-linked sodium carboxymethyl cellulose, and effervescent disintegrants; the non-aqueous solvents comprise a composition of one or more of soybean oil, castor oil, and peanut oil; the solubilizers comprise a composition of one or more of Tween 80, Tween 60, and poloxamer 68; the cosolvents comprise a composition of one or more of sodium benzoate, sodium salicylate, and sodium p-aminobenzoate;
optionally, a mode of administration of the pharmaceutical composition comprises oral administration (e.g., oral cavity), sublingual administration, parenteral administration (e.g., intramuscular, intravenous or subcutaneous administration), rectal administration (e.g., by suppositories or lotions), transdermal administration (skin electroporation, transdermal preparations, etc., such as creams, gels, paints, and transdermal patches) or inhalation administration (e.g., aerosols);
optionally, the pharmaceutical composition is administered in a single unit dose form or in an *ad libitum* single dose under continuous treatment;
optionally, the pharmaceutical composition may further be in the form of an oil emulsion or dispersion in combination with a lipophilic salt such as pamoic acid, or in the form of a biodegradable sustained-release composition for use in subcutaneous or intramuscular administration;
optionally, the pharmaceutical composition may be prepared into a solid oral formulation, a liquid oral formulation, an injection, or a transdermal formulation;
optionally, the solid and liquid oral formulations comprise: tablets, dispersible tablets, sugar-coated tablets, granules, dry powders, capsules, syrups, and solutions;
optionally, the injection comprises: small injections, large volume parenteral, and lyophilized powder injections;
optionally, the transdermal formulation comprises: ointments, plasters, liniments, aerosols, traditional pastes, adhesive dispersion patches, peripheral adhesive skeleton patches, reservoir patches, and cataplasms.

**8.** Use of the compound represented by formula (I), or the optical isomer thereof, or the pharmaceutically acceptable salt thereof, or the solvate thereof (such as a hydrate), or the inclusion compound thereof, or the racemate thereof, or the co-crystal thereof, or the isotopically labelled compound thereof, or the nitrogen oxide thereof according to any one of claims 1 to 5, or the pharmaceutical composition according to claim 7 in the preparation of a medicament for preventing and/or treating inflammation, pain, fever, cancer, Alzheimer's disease, etc.;
optionally, the inflammation is selected from rheumatoid arthritis, osteoarthritis, ankylosing spondylitis, scapulohumeral periarthritis, myotenositis and tenosynovitis, peritendinitis, lateral epicondylitis (tennis elbow), postoperative anti-inflammation, and swelling and inflammation caused by injury;
optionally, the pain is selected from mild to moderate pain, pain after trauma or strain, dysmenorrhea and postoperative pain, toothache and cancer pain, acute pain in adults, etc.;
optionally, the fever comprises fever caused by common cold or influenza;
optionally, the cancer is selected from gastric cancer, esophageal cancer, multiple myeloma, brain glioma, and lung cancer;
optionally, cells of the cancer comprise human esophageal carcinoma cell Eca-109, human multiple myeloma cell MM.1S, human gastric carcinoma cell NUGC-4, human brain astrocyte U87, human glioma cell U251, and large cell lung carcinoma cell H460.

**9.** Use of the compound represented by formula (I), or the optical isomer thereof, or the pharmaceutically acceptable salt thereof, or the solvate thereof (such as a hydrate), or the inclusion compound thereof, or the racemate thereof, or the co-crystal thereof, or the isotopically labelled compound thereof, or the nitrogen oxide thereof according to any one of claims 1 to 5, or the pharmaceutical composition according to claim 7 in combination with an additional one or more second active compounds in the preparation of a medicament for preventing and/or treating inflammation, pain, fever, cancer or Alzheimer's disease;
optionally, the inflammation is selected from rheumatoid arthritis, osteoarthritis, ankylosing spondylitis, scapulohumeral periarthritis, myotenositis and tenosynovitis, peritendinitis, lateral epicondylitis (tennis elbow), postoperative anti-inflammation, and swelling and inflammation caused by injury;
optionally, the pain is selected from mild to moderate pain, pain after trauma or strain, such as dysmenorrhea and postoperative pain, toothache and cancer pain, acute pain in adults, etc.;
optionally, the fever comprises fever caused by common cold or influenza;
optionally, the cancer is selected from gastric cancer, esophageal cancer, multiple myeloma, brain glioma, and lung cancer;
optionally, cells of the cancer comprise human esophageal carcinoma cell Eca-109, human multiple myeloma cell MM.1S, human gastric carcinoma cell NUGC-4, human brain astrocyte U87, human glioma cell U251, or large cell lung carcinoma cell H460;
optionally, examples of the additional one or more second active compounds as active drugs are one or more selected from the following substances: sufentanil, dexmedetomidine, formoterol, isoproterenol, salbutamol, bambuterol, procaterol, fenoterol, arformoterol, tulobuterol, clenbuterol, salmeterol, salmeterol casone, terbutaline, orciprenaline, and chlorprenaline.
